(19) **Europäisches Patentamt / European Patent Office / Office européen des brevets**

(11) **EP 1 226 784 B1**

(12) **EUROPEAN PATENT SPECIFICATION**

(45) Date of publication and mention
of the grant of the patent:
**11.04.2007 Bulletin 2007/15**

(51) Int Cl.:
*A61B 5/00* *(2006.01)*  *A61B 5/0205* *(2006.01)*
*A61B 5/22* *(2006.01)*

(21) Application number: **02001799.2**

(22) Date of filing: **25.01.2002**

(54) **Vital signs detection system and health control method**

System zur Erfassung von Lebenszeichen und Verfahren zur Gesundheitskontrolle

Système de détection de signaux vitaux et méthode de contrôle de santé

(84) Designated Contracting States:
**DE FR GB**

(30) Priority: **25.01.2001 JP 2001017046**

(43) Date of publication of application:
**31.07.2002 Bulletin 2002/31**

(73) Proprietor: **MATSUSHITA ELECTRIC INDUSTRIAL CO., LTD.**
**Kadoma-shi, Osaka 571-8501 (JP)**

(72) Inventor: **Mori, Yasuhiro**
**Izumi-shi,**
**Osaka 594-007 (JP)**

(74) Representative: **Grünecker, Kinkeldey,**
**Stockmair & Schwanhäusser**
**Anwaltssozietät**
**Maximilianstrasse 58**
**80538 München (DE)**

EP 1 226 784 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

The present invention relates to a vital signs detection system and a vital signs detection method for acquiring and processing vital signs from an organism, and a health control method using the same.

Exercise is effective for prevention against and treatment for adult diseases. That is, exercise strengthens the function of the heart, the lungs, and the muscles, resolves fatness, reduces the blood sugar level and the blood pressure, and increases the HDL cholesterol (good cholesterol) component. In particular, for diabetics who are recently increasing in number (95% of diabetes is of non-insulin-dependent diabetes mellitus (NIDDM) in Japan), it is reported that exercise is to be effective in: reduction in the blood pressure; improvement in various conditions of diabetes; prevention of arteriosclerosis; and prevention of aging.

For the purpose of prevention and treatment, recommended exercise is a whole-body exercise causing deep breathing, such as walking, jogging, rope skipping, in-pool walking, swimming, cycling, and aerobics. Recommended intensity of the exercise is 40% to 60% in a scale in which "the hardest" exercise (no harder exercise is possible) is rated at 100%. The intensity of the exercise is measured, for example, according to a subjective symptom (Borg's scale) as shown in Table 1.

[Table 1]

| | |
|---|---|
| 6 | |
| 7 | very, Very Light |
| 8 | |
| 9 | Very Light |
| 10 | |
| 11 | Fairly Light |
| 12 | |
| 13 | Somewhat Hard |
| 14 | |
| 15 | Hard |
| 16 | |
| 17 | Very Hard |
| 18 | |
| 19 | Very, Very Hard |
| 20 | |

As shown in Table 1, a recommended exercise is that in the range of feeling "from Fairly Light to Somewhat Hard."

This measurement method depends on the feeling. Accordingly, the use of a judgment method based on the heart rate is more realistic and safer. A measure of heart rate at exercise is obtained by the following Blackburn's formula which gives the heart rate easily.

$$(\text{heart rate}) \fallingdotseq (220 - (\text{age})) \times K$$

(Here, K indicates exercise intensity of 0<K<1.)

For example, in case of an exercise by a 40-year old patient at an intensity of 50%, the formula gives (220 40) ×0.5=90. This indicates that an exercise causing a heart rate of 90 is recommended.

Such real-time recognition of the heart rate permits the recognition of the intensity of the exercise, and is important in order to take exercise within a range of exercise intensity avoiding over-exercise to one's body.

Further, in order to recognize the effect of an exercise as a concrete value, it is appropriate to measure the calorie consumption. Such real-time recognition of the heart rate and the calorie consumption is very important in exercise for the purpose of prevention against and treatment for adult diseases.

Even for healthy persons, dieting is attracting the attention, as indicated by the fact that the dieting is a popular theme in women's weeklies. Accordingly, the real-time recognition of calorie consumption in daily life encourages the continuation of dieting, and has other various advantages.

Accordingly, for the purpose of health control, apparatuses for measuring the heart rate and the calorie consumption of a user are used in medical scenes, offices, homes, and the like.

An exemplary apparatus for measuring the calorie consumption of a user calculates the calorie consumption on the basis of the heart rate. In some cases, such a consumption calorimeter further comprises a body motion detection apparatus and a body temperature detection apparatus in addition to a pulse wave detection apparatus, and thereby avoids variation due to psychological factors so as to obtain a more precise value of calorie consumption.

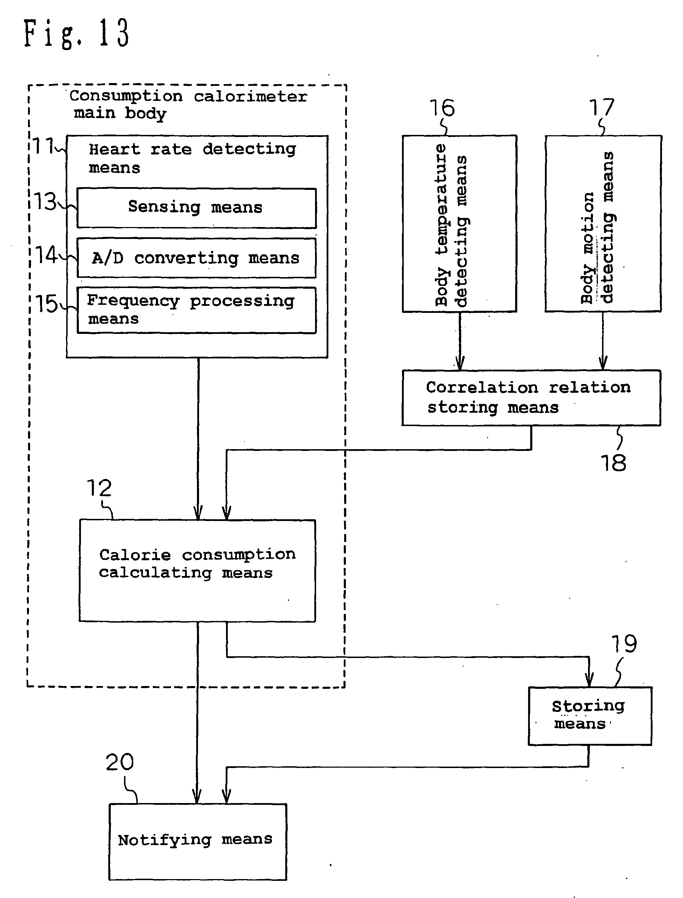

Figure 13 is a block diagram showing the configuration of a prior art consumption calorimeter for calculating calorie consumption on the basis of heart rate. As shown in the figure, fundamental components of this prior art consumption calorimeter are heart rate detecting means 11 and calorie consumption calculating means 12. The heart rate detecting means 11 comprises pulse wave detecting means 13, A/D converting means 14, and a frequency processing means 15. A heart rate detected by the heart rate detecting means 11 is substituted into an approximation formula, whereby the calorie consumption is obtained. Body temperature detecting means 16 and body motion detecting means 17 are also provided. On the basis of the outputs from these means, correlation relation storing means 18 selects an approximation formula used for the calculation of the calorie consumption, so as to improve the precision in the calculation of the calorie consumption. The calorie consumption calculating means 12 substitutes the heart rate into the approximation formula selected by the correlation relation storing means 18, and thereby calculates the calorie consumption. The value of calorie consumption is stored in time series in storing means 19, and at the same time, displayed on notifying means 20 so as to be notified to a user in real time.

The body motion detecting means 17 determines whether the user is moving or not. When the user is found to be moving, an approximation formula for active states is used. Otherwise, an approximation formula for resting states is used. The body temperature detecting means 16 detects the body temperature. On the basis of this body temperature, the condition of fundamental metabolism is identified, or alternatively, the body temperature is used as a parameter in the determination of the activity state of the user. Such a configuration permits a precise measurement (within the error of 5%) of the value of calorie consumption.

Such a consumption calorimeter can be implemented in a wearable form such as a watch shape, a necklace shape, a card shape, and an eyeglasses shape. In case of the watch shape, the heart rate and the calorie consumption are displayed on the LCD (Liquid Crystal Display), of the watch in real time. Further, displayed thereon are statistical data, such as accumulated calorie consumption and the degree of achievement, and graphs illustrating such data.

Nevertheless, such a prior art consumption calorimeter is dedicated to the detection of calorie consumption. Accordingly, the other vital signs cannot be treated. For example, a user who desires to measure the blood pressure in addition to the calorie consumption needs to attach a blood -pressure meter (composed of a section for sensing blood pressure information and a section for processing the data obtained by the sensing) independently in addition to the above-mentioned consumption calorimeter.

A multi-user remote health monitoring system is described in US 6 101 478. The system comprises a server, a workstation and a plurality of remotely programmable, apparatuses connected with monitoring devices. The remotely programmable apparatuses work in accordance with script programs received from the server. The remotely programmable apparatuses transmit an identification code characterising a patient's identification or a patient type (i.e. a specific disease).

EP 0 922 434 describes a system for simultaneous monitoring of a plurality of individuals comprising a computer, a Central Interface Unit having a processor, and a plurality of Individual Physiological Data Loggers (IPDL) including an Electro Cardiogram (ECG) measuring device. Each of the IPDL devices is identified by and ID number that is forwarded to the computer.

As such, with increasing number of the items to be monitored in the organism, measurement instruments (each composed of a section for sensing vital signs corresponding to the monitored item and a section for processing the data) need to be added correspondingly to the increased items.

Further, in case of a secondary use of the data obtained by the instruments, the data is, in some cases, transmitted to an external server for information processing. In this case, the increase in the number of the measurement instruments causes an increase also in the number of hardware pieces for data transfer which need to be provided in each measurement instrument. This results in an complexity in the configuration of the entire system, and also a reduction in workability.

As for a single measurement instrument such as the above-mentioned consumption calorimeter, there have been the following problems.

A first problem is the capability of displaying. In case of a wearable apparatus described above, the display area and the resolution in the display device are restricted. Accordingly, the amount of displayed data and the character size are in trade-off. That is, when small characters are used for display, a large amount of data can be displayed. Nevertheless, this is not appropriate for elderly persons and diabetics who have poor sight in many cases.

A second problem is the operation keys and the operability thereof. Since a wearable device is necessarily small and has a small surface area, the keys provided thereon need to be small, and the number of the keys is also restricted. Accordingly, two or more functions are assigned to a single key in most cases. This degrades the operability.

It is desirable that a device can be operated easily with a small number of keys. Nevertheless, in the prior art devices, real-time measurement data and statistical data derived therefrom have been displayed on a single display device. This has unavoidably caused a large number of key operations, and hence prevented simple key operation.

A third problem is a difficulty in application to users. It is laborious to receive an exercise menu in the form of paper or e-mail and thereby input one's own target values so as to determine the exercise menu of the final form. This can disturb the habit of exercise. Accordingly, it is desirable that an entire menu is automatically downloaded, displayed, and set on the health control apparatus.

The present invention has been devised with considering the above-mentioned problems. An object of the present invention is to provide: a vital signs detection system applicable to various purposes and various types of necessary data depending on the purpose; and a health control method using the same.

This is achieved by the features of the independent claims. Further features and advantages of the present invention are the subject matter of dependent claims.

Figure 1 is a block diagram showing a health control apparatus according to Embodiment 1 of the invention.
Figure 2 is a block diagram showing an example of the mapping in storing means of a health control apparatus.
Figure 3 is a flowchart showing the algorithm in a health control apparatus.
Figure 4 is a block diagram showing another exemplary configuration of a health control apparatus according to Embodiment 1 of the invention.
Figure 5 is a block diagram showing a health controls apparatus according to Embodiment 2 of the invention.
Figure 6 is a block diagram showing another exemplary configuration of a health control apparatus according to Embodiment 2 of the invention.
Figure 7 is a block diagram showing further another exemplary configuration of a health control apparatus according to Embodiment 2 of the invention.
Figure 8 is a block diagram showing the internal configuration of calorie consumption calculating means 143 of a health control apparatus according to Embodiment 2 of the invention.
Figure 9 is a block diagram showing an example of a health control method using a vital signs detection apparatus according to Embodiment 3 of the invention.
Figure 10 is a flowchart showing the algorithm in the example of a health control method shown in Figure 9.
Figure 11 is a diagram showing the operation that a user uploads measurement data in a health control method using a vital signs detection apparatus according to Embodiment 3 of the invention.
Figure 12 is a diagram showing the operation that an instructor uploads measurement data in a health control method using a vital signs detection apparatus according to Embodiment 3 of the invention.
Figure 13 is a block diagram showing the configuration of a prior art consumption calorimeter.
Figure 14 is a configuration diagram of a vital signs detection system according to Embodiment 4 of the invention.
Figure 15 is a configuration diagram of a vital signs detection system according to Embodiments 5 and 6 of the invention.
Figure 16 is a flowchart showing the operation of a vital signs detection system according to Embodiment 5 of the invention.
Figure 17 is a flowchart showing another example of the operation of a vital signs detection system according to Embodiment 5 of the invention.
Figure 18 is a flowchart illustrating the operation of a vital signs detection system according to Embodiment 6 of the invention.

Description of the Reference Numerals

| | |
|---|---|
| 102-104 | Sensing apparatus |
| 105-107 | Sensing means |
| 108-110 | A/D converting means |
| 111-113 | Buffering means |
| 114-116 | Communicating means |
| 121 | Data processing apparatus |
| 122 | Communicating means |
| 123 | Data processing means |

| 124 | Displaying means |
| 125 | Storing means |
| 130 | Information server |
| 140 | Program server |

The embodiments of the present invention are described below with reference to the drawings.

(Embodiment 1)

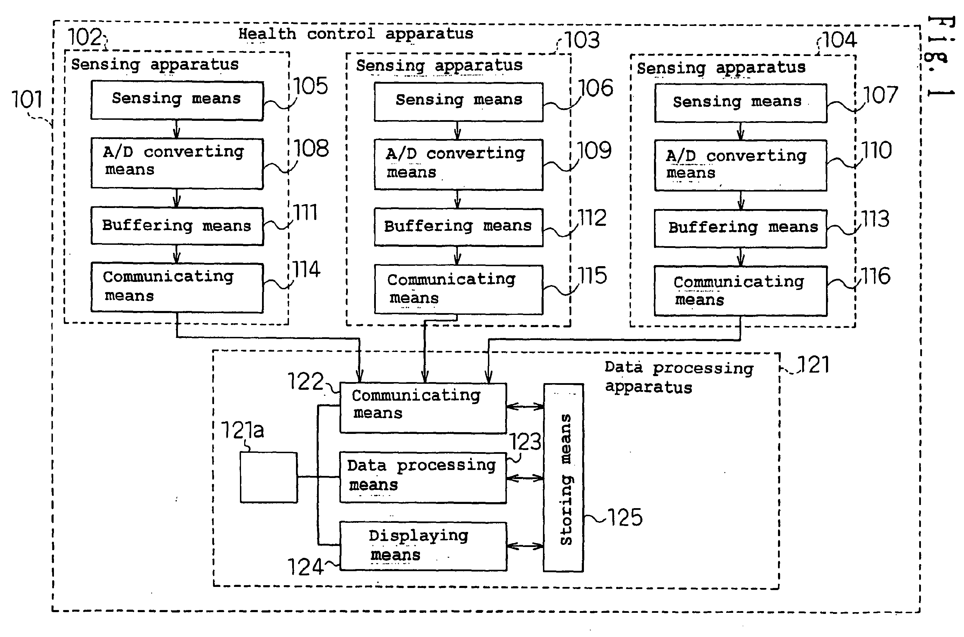

Figure 1 is a block diagram showing a health control apparatus according to Embodiment 1 of the invention. As shown in the figure, the health control apparatus 101 comprises one or more sensing apparatuses and a data processing apparatus. Illustrated in Figure 1 is an exemplary health control apparatus comprising three sensing apparatuses 102-104 and a data processing apparatus 121.

Each sensing apparatus 102-104 comprises: each sensing means 105-107 of detecting (sensing) vital signs; each A/D converting means 108-110 of converting the detected an analog signal into a digital signal; each buffering means 111-113 of temporarily storing the vital data which is digital-converted vital signs; and each communicating means 114-116. The data processing apparatus 121 comprises: communicating means 122; data processing means 123 of processing the data of vital data and the like; displaying means 124 of displaying the data of vital data and the like; and storing means 125 of storing the data of vital data and the like. In the above-mentioned configuration, the health control apparatus 101 is an example of a vital signs processing apparatus according to the invention. Each sensing apparatus 102-104 is an example of vital signs detecting means according to the present invention. The data processing apparatus 121 is an example of vital signs processing means according to the present invention. These correspondences are the same also in the embodiments described below.

The communicating means 114-116 of the sensing apparatuses 102-104 can communicate with the communicating means 122 of the data processing apparatus 121. According to the specification, the communicating means 114-116 cannot communicate with each other, or alternatively, these communicating means perform solely a relaying operation of transferring the data transmitted from an adjacent sensing apparatus, intact to the communicating means of another sensing apparatus or the data processing apparatus. These communicating means do not write the received data into the buffering means 111-113.

The data processing means 123 is composed of either dedicated hardware or a programmable device provided with a CPU, and thereby processes vital signs and the like stored in the storing means 125. In case that the data processing means is composed of a programmable device, the function of the data processing means 123 is implemented by software which is stored in the storing means 125.

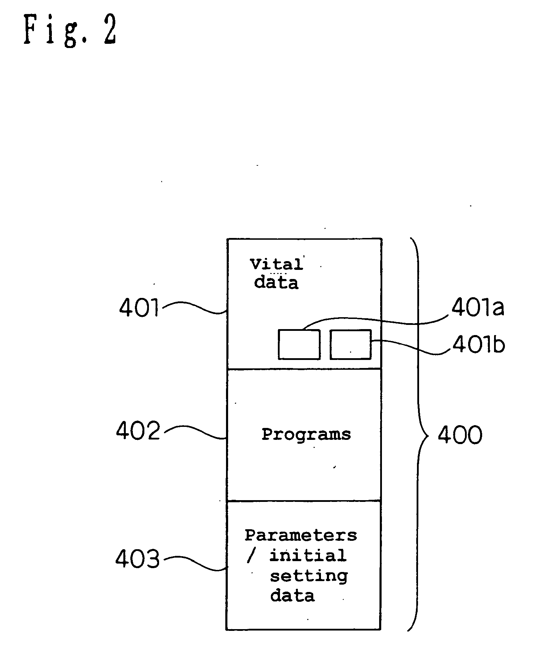

The storing means 125 is a device capable of data reading and writing. As shown in Figure 2, a storage region 400 of the storing means 125 is divided into: a region 401 for storing the vital data in time series; a region 402 for storing the program for the data processing means; and a region 403 for storing the parameters and the initial setting data necessary for the execution of the data processing means. The vital data region 401 is further divided into: a measurement data region 401a for storing the data measured by the sensing apparatuses 102-104; and a processing data region 401b for storing the processing result from the data processing means 123.

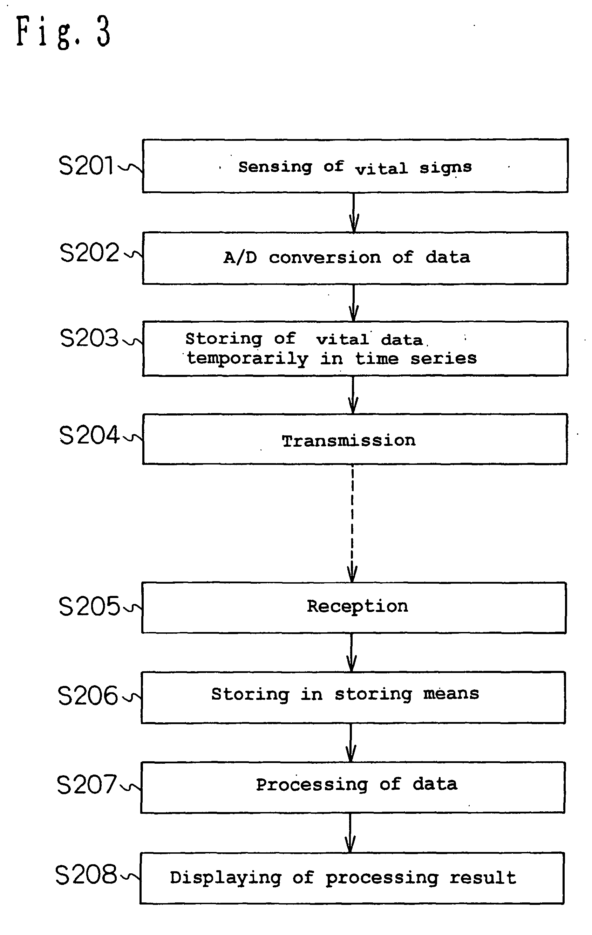

Figure 3 is a flowchart explaining the operation of the health control apparatus according to the present embodiment.

The operation of the health control apparatus according to the present embodiment is described below with reference to Figure 3.

Each sensing apparatus 102-104 repeats the following operation.

(S201) Each sensing means 105-107 detects (senses) the vital signs of a user. (S202) Each A/D converting means 108-110 converts the vital signs obtained by each sensing means 105-107 into digital vital data. (S203) Each buffering means 111-113 stores the vital data in time series in first-in first-out scheme. (S204) Each communicating means 114-116 reads out the vital data from each buffering means 111-113, and then transmits the data to the data processing apparatus 121.

During the above-mentioned operation, the sensing of each sensing means 105-107 in (S201) may be invoked by a demand from the data processing apparatus 121 (on-demand type). Alternatively, the sensing may be carried out periodically with a period predetermined by the data processing apparatus 121 (periodical execution type). Further, the sensing may be of continuous measurement type. These types are selectable depending on the item to be measured. As described later, the measurement of pulse wave is carried out according to the continuous measurement type, while the measurement of body temperature is carried out according to the periodical execution type or the on-demand type. Similarly, in (S204), the data transmission of each communicating means 114-116 may be carried out in response to a data transmission request from the data processing apparatus 121, or alternatively, may be carried out spontaneously and periodically. Alternatively, the transmission may be carried out continuously as long as vital data exists in each buffering means 111-113. In any way, the sensing operation in (S201) and the transmission operation in (S204) do not need to be carried out in synchronization with each other, and may be carried out completely asynchronously.

Described below is the operation of the data processing apparatus 121.

(S205) The communicating means 122 receives the vital data from each sensing apparatus 102-104. (S206) The communicating means 122 writes the received vital data into the storing means 125. (S207) The data processing means 123 processes the vital data stored in the storing means 125, and then stores the processing result into the processing data region 401b within the region 401 for storing the vital data within the storing means 125. (S208) The processing result is read out and displayed on the displaying means 120.

Each of the three sensing apparatuses 102-104 in the description of the present embodiment may measure an item (for example, body temperature, blood pressure, blood sugar level, and the like) different from that of the other sensing apparatuses. Alternatively, these sensing apparatuses may measure the same item.

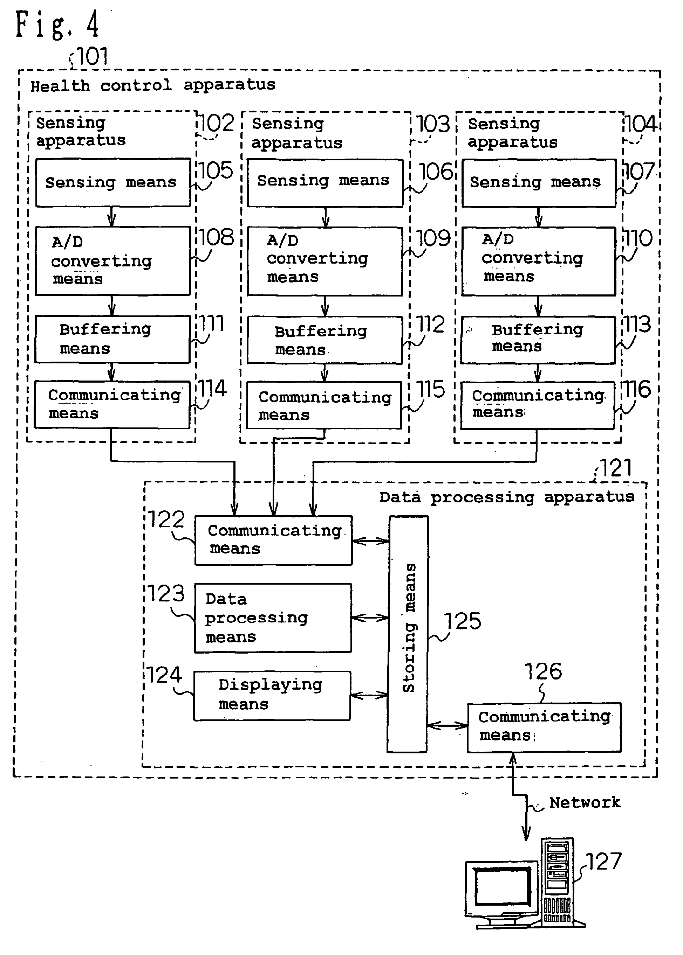

Figure 4 is a block diagram showing another exemplary configuration of a health control apparatus according to the present embodiment. The health control apparatus shown in Figure 4 further comprises, in addition to the configuration shown in Figure 1, communicating means 126 for connecting the data processing apparatus 121 to a network and thereby communicating with an external server 127.

The communicating means 126 implements the communication with the server 127 by wire or wireless. The communicating means 126 permits: the upload of the data stored in the storing means 125, to the server 127; and the download of the parameters or even the program itself used for the operation of the data processing means 123, from the server 127.

According to this configuration, the vital data acquired by the health control apparatus is stored in the external server 127. This permits the long-term management of the health condition of the user of the health control apparatus. In case that the user utilizing or managing the server 127 is a doctor/health control service provider providing doctor/health control services, when the programs and/or parameters necessary for the health control are stored in the server 127 and then downloaded to the data processing apparatus 121 as described above, the health control instruction for the user is carried out more easily.

In Figure 1, each sensing apparatus 102-104 and the data processing apparatus 121 exchanges the data by wire or wireless communication through communicating means 114-116, 122. Further, in another exemplary configuration of the health control apparatus, each communicating means 114-116, 122 may be composed of an interface for a removable medium, and each of the buffering means 111-113 and the storing means 125 may be composed of the removable medium.

In this configuration, the user attaches the sensing apparatus to his/her body for a predetermined time, and thereby stores the vital signs in the removable medium. The length of the time is determined depending on the type of the measurement item, within the maximum value calculated by the formula: (the capacity of the removable medium) / (the amount of data per unit time). After the predetermined time, the user removes the removable medium from the sensing apparatus, and then inserts the removable medium into the data processing apparatus. Then, the data processing apparatus reads the vital data from the removable medium, and then performs necessary processing onto the organism data.

In the configuration shown in Figure 4, the storing means 125 may be a removable medium, while the communicating means 126 may be interface means for the removable medium. In this configuration, when the user uploads the data stored in the removable medium to the server, the user removes the removable medium, and then inserts the removable medium into the read-out apparatus. The read-out apparatus may be connected to the server directly, or alternatively, via a network. The server can write the personal data, the health control indices, and the exercise indices into the removable medium, and thereby set these data into the health control apparatus.

The type of the removable medium may be selected depending on the item to be measured, but a nonvolatile semiconductor memory is generally the most appropriate medium.

(Embodiment 2)

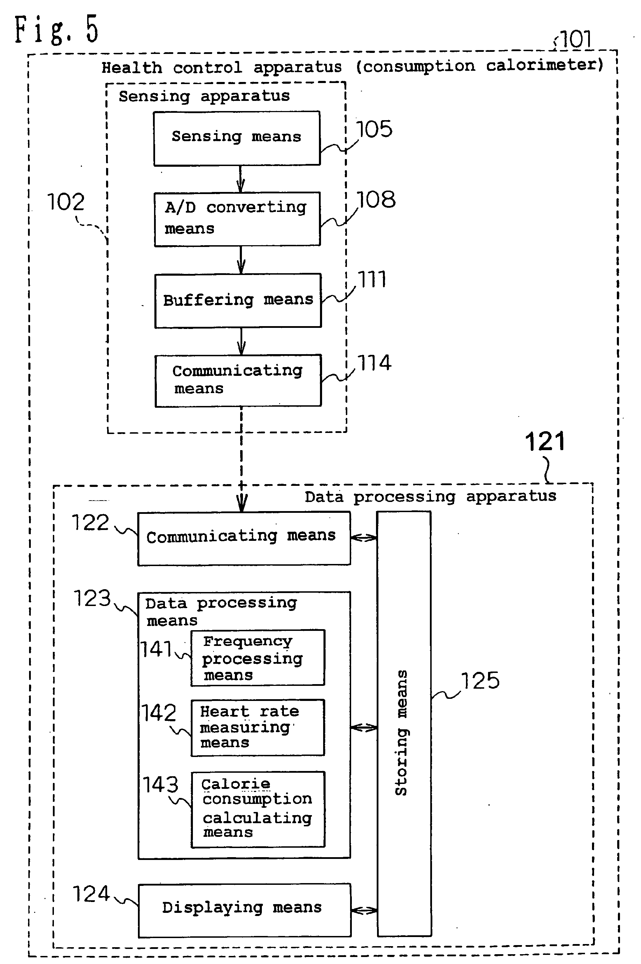

Figure 5 is a block diagram showing a consumption calorimeter according to Embodiment 2 of the invention. The consumption calorimeter shown in Figure 5 is an exemplary modification of the health control apparatus according to Embodiment 1.

In the consumption calorimeter according to the present embodiment, the data processing means 123 comprises frequency processing means 141, heart rate measuring means 142, and calorie consumption calculating means 143. The sensing means 105 of the sensing apparatus 102 is composed of a pulse wave sensor for detecting the pulse wave. In Figure 5, a single sensing apparatus is illustrated, however, two or more sensing apparatuses may be included in the system. In this case that two or more sensing apparatuses are attached, even when the data cannot be received from one sensing apparatus, the calorie consumption can be calculated from the data from the other sensing apparatuses. Alternatively, when the pulse wave data from these sensing apparatuses are averaged out, the reliability in the calculated calorie consumption is improved.

Next, the data processing means 123 comprises frequency processing means 141, heart rate measuring means 142 , and calorie consumption calculating means 143. The frequency processing means 141 performs FFT (fast Fourier transformation) processing onto the pulse wave data acquired by the sensing apparatus. The heart rate measuring means 142 obtains the heart rate from the processing result. On the basis of the heart rate, the calorie consumption calculating means 143 calculates the calorie consumption in each unit time. Similarly to Embodiment 1, the function of the frequency processing means 141, the heart rate measuring means 142, and the calorie consumption calculating means 143 may be implemented by dedicated hardware, or alternatively, by a program (software) executed on a programmable device having a CPU core. In case that the software is used, the storage regions for program, stack, and variables are prepared in the storing means 125.

Described below is the operation of the consumption calorimeter having the above-mentioned configuration according to the present embodiment.

In the sensing apparatus 102, the sensing means 105 composed of a pulse wave sensor detects the pulse wave of the user. The pulse wave is A/D-converted by the A/D converting means 108. Then, the pulse wave data of digital nature is transmitted to the data processing apparatus 121. The communicating means 122 receives the pulse wave data, and then stores the data in time series into the storing means 125. In the data processing means 123, the frequency processing means 141 performs FFT processing onto the pulse wave data stored in the storing means 125. The heart rate measuring means 142 obtains the heart rate from the processing result. On the basis of the heart rate, the calorie consumption calculating means 143 calculates the calorie consumption in each unit time. The calculated calorie consumption is stored in a region different from that storing the pulse wave data within the storing means 125, and then ready for being displayed on displaying means 124.

Regarding the above-mentioned operation, it has been reported that the calorie consumption and the heart rate are highly correlated, and that the calorie consumption can be calculated approximately from the heart rate. Accordingly, the heart rate is substituted into a predetermined approximation formula, whereby the calorie consumption is calculated. As such, in the operation of the data processing means 123, the calorie consumption is calculated on the basis of the above-mentioned correlation.

In an example, the calorie consumption is calculated according to the following formula.

$$\texttt{calorie consumption (kcal)} = \texttt{heart rate at exercise}$$
$$\texttt{(pulse/min)} \times \texttt{time (min)} \times \texttt{constant a}$$

Here, the constant a is obtained by measuring the constant running speed at 150 m/min and the heart rate and by utilizing the following formula.

$$\texttt{constant a} = 0.193 \times 0.005 \times \texttt{running speed (m/min)}$$
$$\times \texttt{body weight (kg) / heart rate (pulse/min)}$$

In the data processing means 123, a measured heart rate is substituted into the above-mentioned formula for calorie consumption, whereby the calorie consumption is obtained.

In the above-mentioned operation, the heart rate may be obtained from the result of the FFT processing onto the pulse wave data as described above. In contrast to this, the peak interval of the pulse wave in the pulse wave data may be obtained, whereby the heart rate is obtained from the inverse thereof.

Using the output of the FFT processing, variation due to psychological factors can be avoided. This permits a more precise calculated value of calorie consumption.

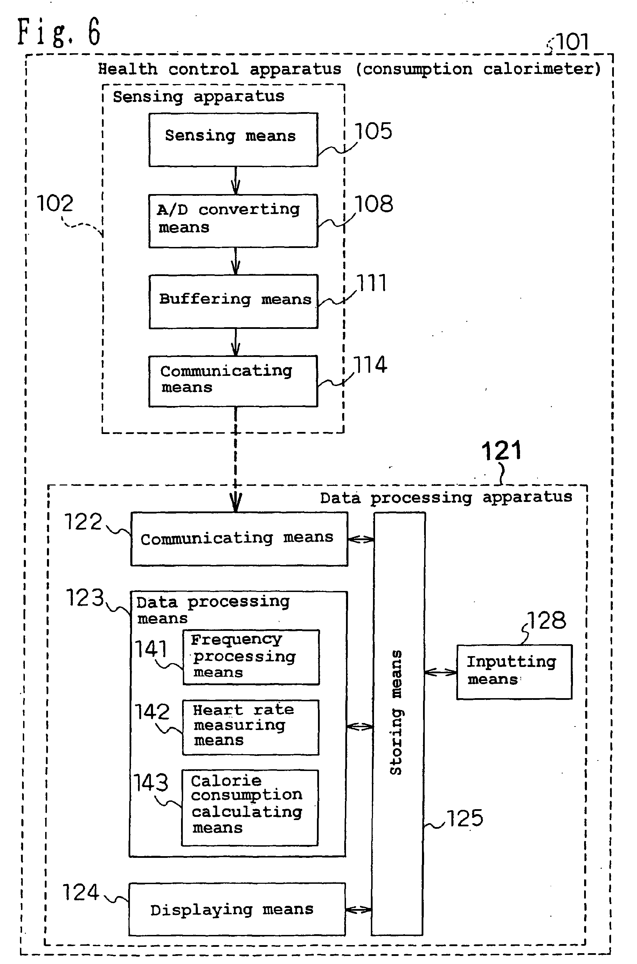

Figure 6 is a block diagram showing another configuration of a consumption calorimeter according to the present embodiment . The consumption calorimeter shown in Figure 6 further comprises, in addition to the configuration shown in Figure 5, inputting means 128 for permitting the user to input the data. The inputting means 128 may be composed of: a pointing device, such as a keyboard, a mouse, and a tablet; or a voice inputting apparatus; generally used in personal computers.

Using the inputting means 128, the user inputs the following data. (1) Personal data...the user's personal information, such as the name, the sex, and the birth date. (2) Health control indices...information on the calorie consumption values per various predetermined unit times, such as daily, weekly, monthly, and final target values for calorie consumption. (3) Exercise indices...record information, such as upper and lower limits for the user's heart rate at exercise and exercise time. And the like.

These data are stored in the storing means 125. When the user takes exercise using the consumption calorimeter, the health control indices and the exercise indices are displayed on the display apparatus 124, whereby the intensity and the time of the exercise are determined with considering these indices.

The inputting means 128 may be detachable from the health control apparatus 101 serving as the consumption calorimeter. That is, the inputting means can be connected to the data processing apparatus of the consumption calorimeter only when the data input is necessary. After the data input, the inputting means can be removed. This configuration does not degrade the portability of the main body of the consumption calorimeter.

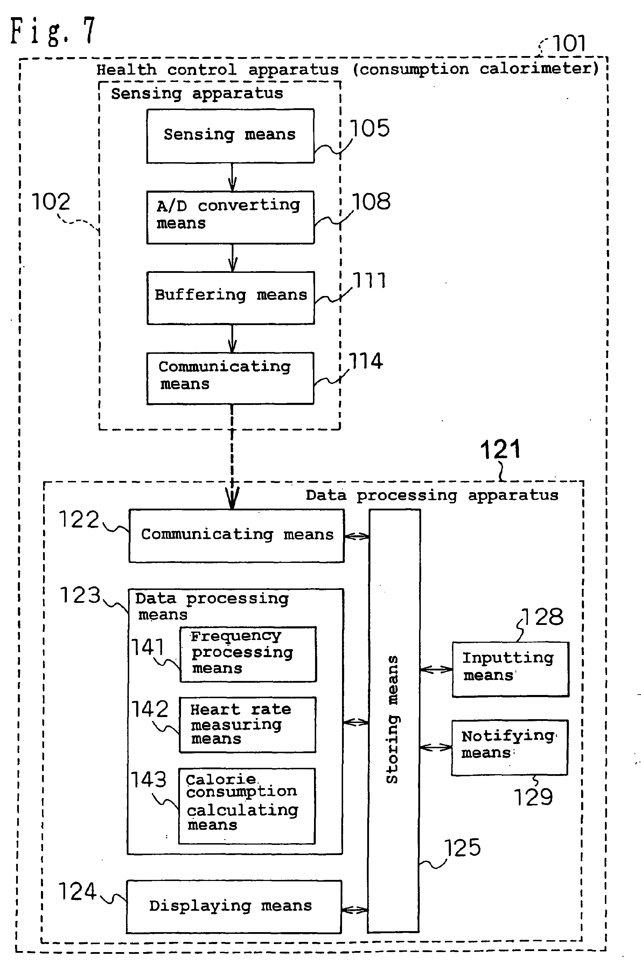

Figure 7 is a block diagram showing further another configuration of a consumption calorimeter according to the present embodiment. The consumption calorimeter shown in Figure 7 further comprises, in addition to the configuration shown in Figure 6, notifying means 129 of notifying information to the user.

The operation of such a consumption calorimeter is as follows. At each measurement of the heart rate, the heart rate measuring means 142 compares the measured heart rate with the upper and lower limits for the heart rate being input through the inputting means 128. When the measured heart rate falls outside the range between the upper and lower limits being input, the heart rate measuring means 142 issues a request for displaying the situation, to the displaying means 124, and at the same time, sends a signal to the notifying means 129. In response to the signal, the notifying means 129 warns the user. The medium of warning information used for warning may be a voice, vibration, light, and any other thing as long as it causes the user to watch the display apparatus 124 of the consumption calorimeter. That is, the notifying means 129 may be implemented by a lamp, an LED, a speaker, a piezoelectric buzzer, a liquid crystal display, a vibrator, and the like.

Receiving the warning information from the notifying means 129, the user can determine the condition of one's own body, and accordingly decreases or increases the exercise intensity. This avoids over-exercise and hence a damage to the body. Thus, the use of the consumption calorimeter ensures the safety in exercise continuously.

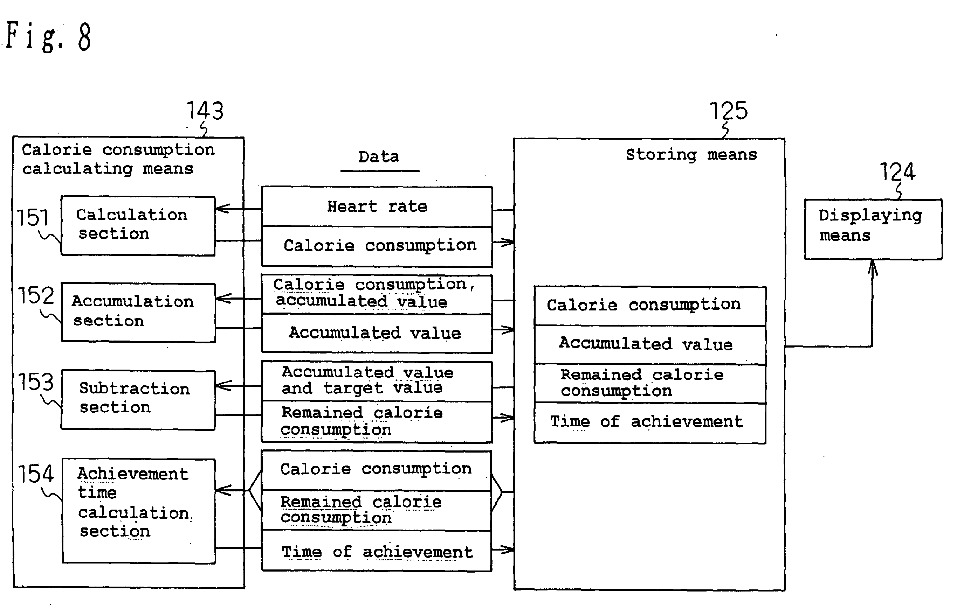

Figure 8 is a block diagram showing the internal configuration of the calorie consumption calculating means 143 of the consumption calorimeter which is an example of a health control apparatus according to the present embodiment. In Figure 8, the calorie consumption calculating means 143 comprises: a calculation section 151 for calculating calorie consumption; an accumulation section 152 for accumulating the calculated calorie consumption; a subtraction section 153 for subtracting the accumulated value from a target value; and an achievement time calculation section 154 for calculating the time for achieving the target value at the current pace of calorie consumption.

The operation is described below. The calculation section 151 reads the heart rate, and then calculates the calorie consumption per unit time according to the formula based on the above-mentioned correlation. The calorie consumption value is written into the processing data region 401b within the storing means 125.

The accumulation section 152 reads the accumulated value and the calorie consumption value from the storing means 125, adds these values to each other, and then writes the obtained sum as the new accumulated value into the processing data region 401b within the storing means 125. At this time, the obtained accumulated value is not overwritten but stored in time series.

The subtraction section 153 reads the accumulated value (the sum between the preceding accumulated value in the time series and the newly obtained calorie consumption value) and the target value, and then calculates the difference between these values. The difference is written as the remained calorie consumption value into the storing means 125.

Finally, the achievement time calculation section 154 divides the remained calorie consumption value obtained by the subtraction section 153, by the calorie consumption value per unit time, and thereby obtains the time necessary for consuming the remained calorie consumption.

In the above-mentioned operation, these data are read out from the storing means 125 and thereby displayed on the displaying means 124 always even during the calculation. Accordingly, the user can recognize these data during the exercise in real time through the displaying means 124. This allows the user to recognize the time of exercise to be continued and the exercise intensity. This encourages the user to continue the exercise. Further, this gives an indicator for an appropriate exercise intensity, and accordingly avoids over-exercise. Thus, the use of the consumption calorimeter ensures the safety in the exercise.

(Embodiment 3)

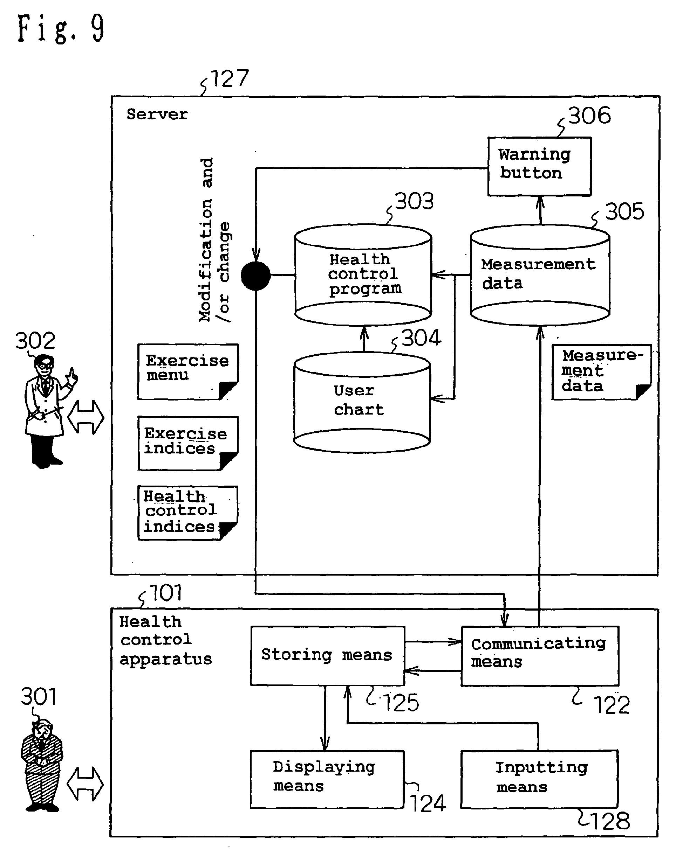

Figure 9 is a diagram explaining a health control method using a vital signs detection apparatus according to Embodiment 3 of the present invention. The example used here of the vital signs detection apparatus of the present invention is the health control apparatus according to Embodiment 1. However, for simplicity, the part of a data processing apparatus is solely illustrated, and the part of a sensing apparatus is omitted. A user 301 is, for example, a patient in hospital or under treatment, and uses a health control apparatus 101.

The health control apparatus 101 is connected to a server 127 via a network such as the Internet. On the server 127 side, there is a health control instructor 302 (referred to as an instructor, hereafter) such as a doctor who instructs the health control of the user 301.

The instructor 302 has a user chart 304 which can be processed as accumulated data in the server 127. Described in the user chart is vital signs, such as the case history (including the present condition), the height, the weight, the body fat percentage, the temperature, and the like of the user.

On the basis of the user chart 304 and the measurement data 305 which is accumulated in the server 127, the server 127 automatically generates the user's health control program 303 which is accumulated in the server 127. However, this is for the case that there is no problem or abnormality in the measurement data. When an abnormality is detected in the measurement data, the instructor 302 manually modifies the health control program.

The health control program 303 is composed of exercise indices, exercise menu, and health control indices. The user 301 takes exercise according to the exercise menu specified there. The intensity and the time to be used in the exercise are specified in the exercise indices. The values specified in the health control indices are used as target values. The health control program 303 contains the items to be carried out in a cycle of a short time (for example, a week or 10 days). This program is a subset extracted from a program which has been specified for a long term, such as one year, by the instructor at the beginning of the health control. Accordingly, when the measurement data in the preceding cycle falls within a predicted range, the items to be carried out in the next cycle are simply extracted.

The user 301 downloads the health control program 303 into the health control apparatus 101. With referring to the program through the displaying means 124, the user takes exercise according to the exercise menu and the exercise indices. During the exercise, the health control apparatus 101 acquires the vital signs from the user 301, and thereby stores the data as the measurement data temporarily in the storing means 125.

After the exercise, the user 301 uploads the measurement data through the communicating means 122 to the server 127.

The above-mentioned operation is repeated, and the execution cycles of the health control program are repeated, whereby the health control of the user is carried out.

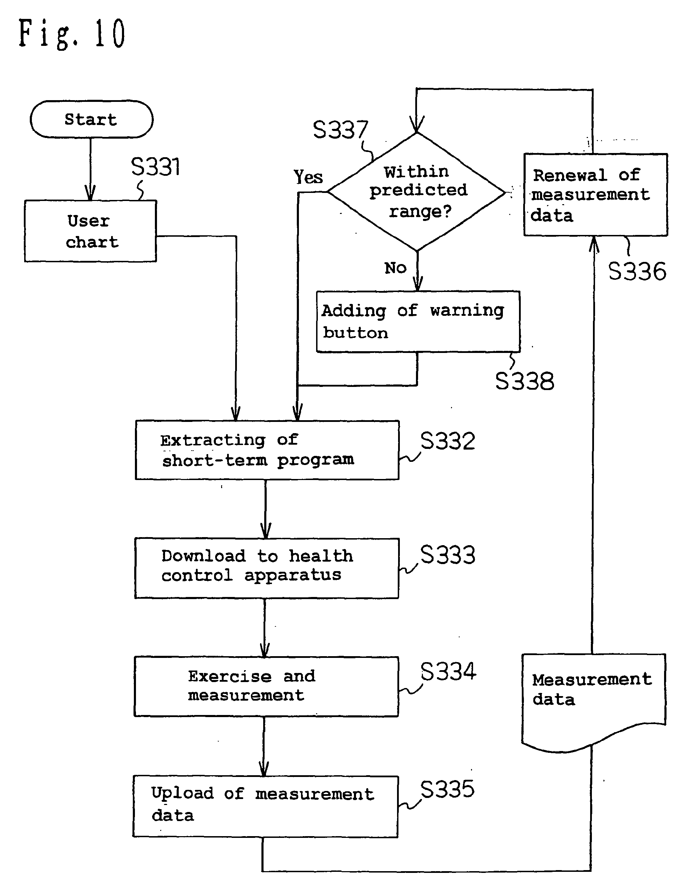

Figure 10 is a flowchart showing the health control method according to the present embodiment. The algorithm is described below with reference to Figure 10.

(S331) The instructor 302 refers to the user chart 304, and thereby determines a health control program 303 based on of the current condition.

(S332) A short-term program used for a short term is extracted from the health control program. At this time, when the measurement data is accompanied with the display of a warning button, the instructor 302 changes the instruction in order to reduce the exercise load, for example, by reducing the amount of exercise, reducing the intensity of exercise, or shortening the time of exercise.

(S333) The health control apparatus 101 downloads the above-mentioned health control program 303 from the server 127 via the network.

(S334) The user 301 takes exercise according to the health control program 303 displayed on the displaying means 124 of the health control apparatus 101. During the exercise, the health control apparatus 101 measures the vital signs of the user 301, and thereby obtains the measurement data.

(S335) The health control apparatus 101 uploads the measurement data to the server 127.

(S336) The server 127 renews the measurement data 305 on the basis of the measurement data uploaded from the health control apparatus 101.

(S337) The server 127 determines whether the measured value obtained from the measurement data falls within the range predicted by the health control program or not. Here, the server 127 comprises determining means of determining this, even though it is not shown in the figure.

(S338) When the measured value falls outside the range predicted by the health control program, the measurement data is displayed with being accompanied with the display of a warning button on the display of the server 127. When the value falls within the range, no warning button is displayed.

With repeating the above-mentioned steps, the health control program 303 is renewed, and the health control of the user 301 is carried out.

In normal cases that no problem occurs in the measurement data, the health control program is renewed automatically. In case that there is a problem, a determination by the instructor is necessary. In this case, the server displays a warning button for warning the user, together with the measurement data.

According to the present embodiment, the instructor 302 on the server 127 side can easily determine whether the measurement data uploaded from the health control apparatus 101 of the user 301 falls within the range of the predicted achievement by the currently used health control program or not. Thus, the instructor 302 can determine either that the use of the current health control program is to be continued, or that a new health control program is to be prepared.

Further, when the measurement data is displayed on the server 127 side, and when the measurement data has the date and time before a predetermined value, a message is transmitted to the health control apparatus 101 so as to request the user 301 to continue the execution of the health control program.

The message transmitted to the health control apparatus 101 may be an electronic text document. When the server 127 and the health control apparatus 101 are connected to each other via the Internet, and when an e-mail address for the user 301 is assigned to the communicating means 125 of the health control apparatus 101, the message can be transmitted as an e-mail to the e-mail address of the user 301 so as to request the user to continue the execution of the health control program.

Instead of the electronic text document, an HTML document may be used as the message, whereby the message is displayed on the displaying means of the health control apparatus of the user so as to request the user to continue the execution of the health control program.

In another example, without using the health control apparatus 101 but with using another method, the server 127 may request the user 301 to continue the execution of the health control program. For example, a terminal capable of processing e-mails, such as a personal computer and a portable phone of the user, may be used. Further, the electronic text document may be transmitted to a facsimile machine owned by the user. In this case, even when the user does not have the habit of frequently checking the reception of e-mails, extra delay is avoided in the notification to the user.

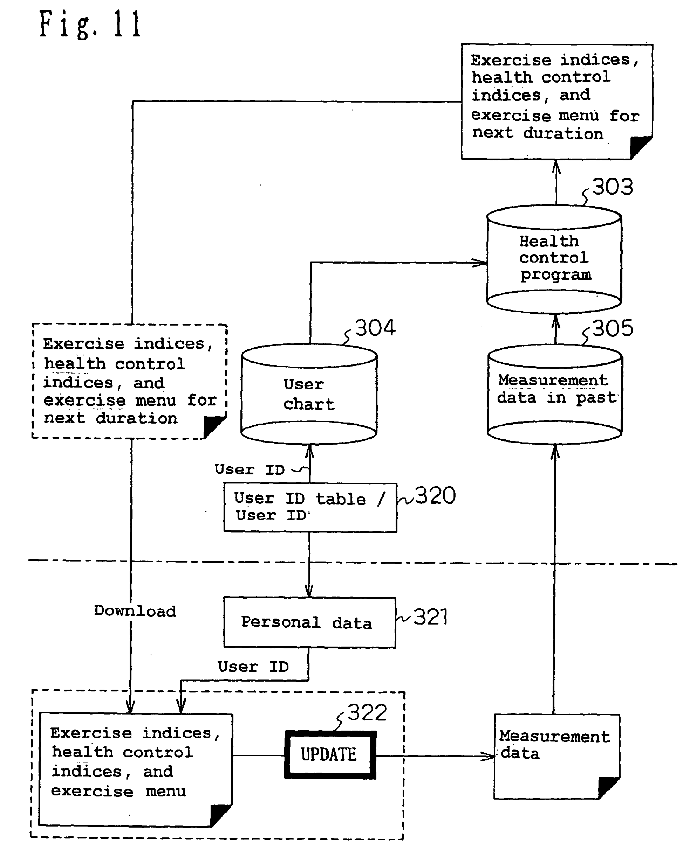

Figure 11 is a diagram showing a health control method according to the present embodiment, in which the user 301 actively uploads the measurement data to the server 127, then downloads a health control program for the next cycle based on the result of the measurement data, from the server 127, and thereby sets the program to the health control apparatus 101. The difference from the example shown in Figure 9 is that the server 127 comprises a user ID table 320 for storing the user ID assigned to the user 301.

In this configuration, when the health control apparatus 101 is initially connected to the server 127, the server 127 assigns a user ID and registers the user ID to the user ID table 320. In the server 127, the user ID is used for corresponding all data of the user 301 to this user.

The server 127 downloads, to the health control apparatus 101, display information containing: a generated health control program; the user ID; and an update button 322 for displaying the operation to be carried out by the user in order to upload the measurement data.

At this time in the operation of the server 127, the health control apparatus 101 does not yet start measurement. Then, the health control apparatus 101 repeats the following steps.

The health control apparatus 101 receives the display information, and then stores the health control program and the user ID together with the personal data 321 into the storing means 122. The displaying means 124 displays the display information containing the update button 322. Then, the user 301 takes exercise on the basis of the exercise menu, the exercise indices, and the health control indices displayed on the displaying means 124, and thereby acquires measurement data.

After the exercise, the user operates the update button 322. As a result of this operation, the measurement data and the user ID are automatically uploaded to the server 127.

In response to the upload of the measurement data, the server 127 renews the contents of the measurement data 305. On the basis of this data and the user chart 304, the server 127 renews the health control program 303, and then prepares a health control program to be used by the user in the next duration.

In this preparation of the health control program, for example, a long-term program previously prepared is adjusted, for example, such that the exercise intensity is weakened or that the exercise time is expanded depending on the measurement data. And then, a health control program for the next duration is extracted. The prepared health control program is downloaded as display information containing the user ID and the update button 322, and then displayed on the health control apparatus of the user.

In the present embodiment, in response to the operation of the update button on the displaying means 124 by the user 301, the inputting means 128 goes ON. The user uploads the measurement data, and then obtains the health control program for the next duration. Accordingly, this embodiment is suitable for the case that a user 301 actively carries out health control.

The display information downloaded from the server 127 may be in HTML format, or alternatively, in XML format.

The display by the update button 322 is that using a standard input device such as a mouse and a keyboard. Conveniently and preferably to the user 301, this display is composed of a single action such as a clicking of an icon.

When the inputting means 128 is composed of a touch panel integrated with the displaying means 124, the input operation may be carried out by touching a specific region in the displaying means 124 by a finger, a pen, or the like. Such a method is visually understandable, and hence easily accepted by various persons.

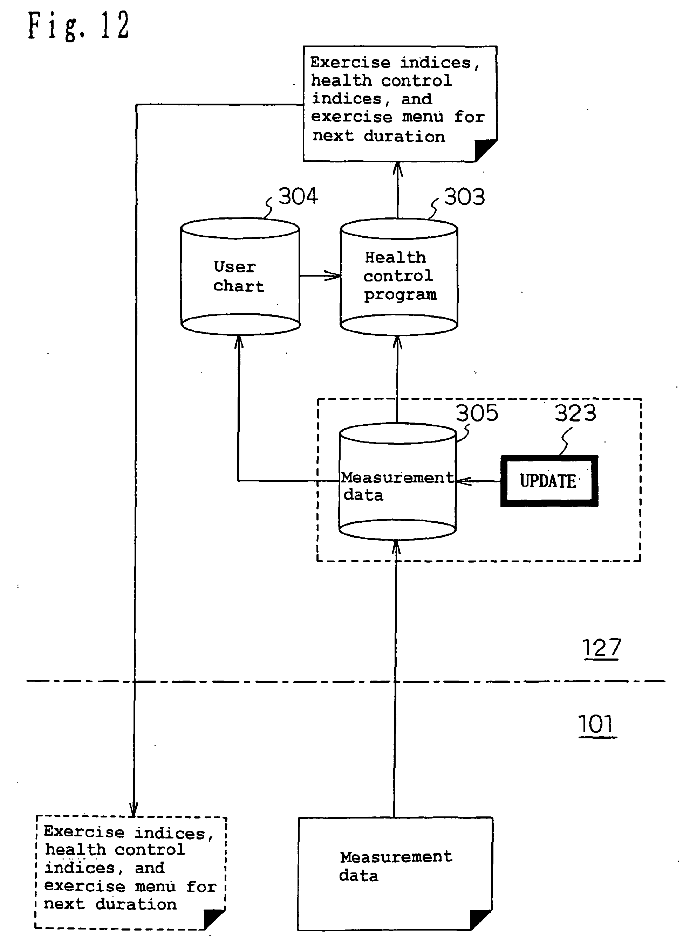

Figure 12 is a diagram showing a health control method according to the present embodiment, in which the instructor 302 actively uploads the measurement data to the server 127, and then downloads a new health control program based on the result of the measurement data, to the health control apparatus. Similarly to the case of Figure 11, the difference from the example shown in Figure 9 is that the server 127 comprises a user ID table for storing the user ID assigned to the user 301.

In this configuration, the server 127 generates display data containing the user ID, the latest measurement data 305, and the date and time of renewal thereof, and then displays the data on a display not shown. When the date and time of renewal has been elapsed for a predetermined time or longer, the server determines that the execution of the health control program is delayed, and thereby adds an update button 323 in the display.

The instructor 302 watches the display data on the display. When an update button 323 is displayed, the instructor operates the button, and thereby renews the measurement data from the health control apparatus 101 corresponding to the user ID corresponding to the update button 323 added in the display.

In response to this renewal operation, when measurement data having the date and time of renewal within the predetermined duration is obtained, the health control program 303 is renewed on the basis of the measurement data, whereby a new health control program for the next duration is generated and downloaded to the health control apparatus 101 corresponding to the user ID.

According to a series of the above-mentioned operations, the measurement data is renewed on the basis of the initiative of the instructor 302. This permits the management of the state of the health control programs of all users 301 of the health control apparatuses 101 connected to the server 127.

Despite the renewal operation, when measurement data having the date and time of renewal within the predetermined duration is not obtained, a warning message can be sent to the user. The message transmitted to the health control apparatus 101 is, for example, an electronic text document. When the server 127 and the health control apparatus 101 are connected to each other via the Internet, and when an e-mail address for the user 301 is assigned to the communicating means 125 of the health control apparatus 101, the message can be transmitted as an e-mail to the e-mail address of the user 301 so as to request the user to continue the execution of the health control program.

Instead of the electronic text document, an HTML document may be used as the message, whereby the message is displayed on the displaying means of the health control apparatus of the user so as to request the user to continue the execution of the health control program.

In another example, without using the health control apparatus 101 but with using another method, the server 127 may request the user 301 to continue the execution of the health control program. For example, a terminal capable of processing e-mails, such as a personal computer and a portable phone of the user, may be used. Further, the electronic text document may be transmitted to a facsimile machine owned by the user. In this case, even when the user does not have the habit of frequently checking the reception of e-mails, extra delay is avoided in the notification to the user. The message may be an electronic text document in the form transmitted as an e-mail to the mail address of the user, or alternatively, in the form transmitted to the facsimile machine owned by the user. Further, the message may be an HTML document in the form displayed on the displaying means of the health control apparatus.

The display by the update button 323 is that using a standard input device such as a mouse and a keyboard. Conveniently and preferably to the instructor 302, this display is composed of a single action such as a clicking of an icon.

When the input device is composed of a touch panel integrated with the display, the input operation may be carried out by touching a specific region on the display by a finger, a pen, or the like. Such a method is visually understandable, and hence easily accepted by various persons.

The display information displayed in the server 127 may be in HTML format. Further, the user ID may be corresponded to the URL of the health control. In this case, by clicking the user ID, a connection is established to the health control apparatus 101 of the user 301, whereby the measurement data is uploaded.


(Embodiment 4)

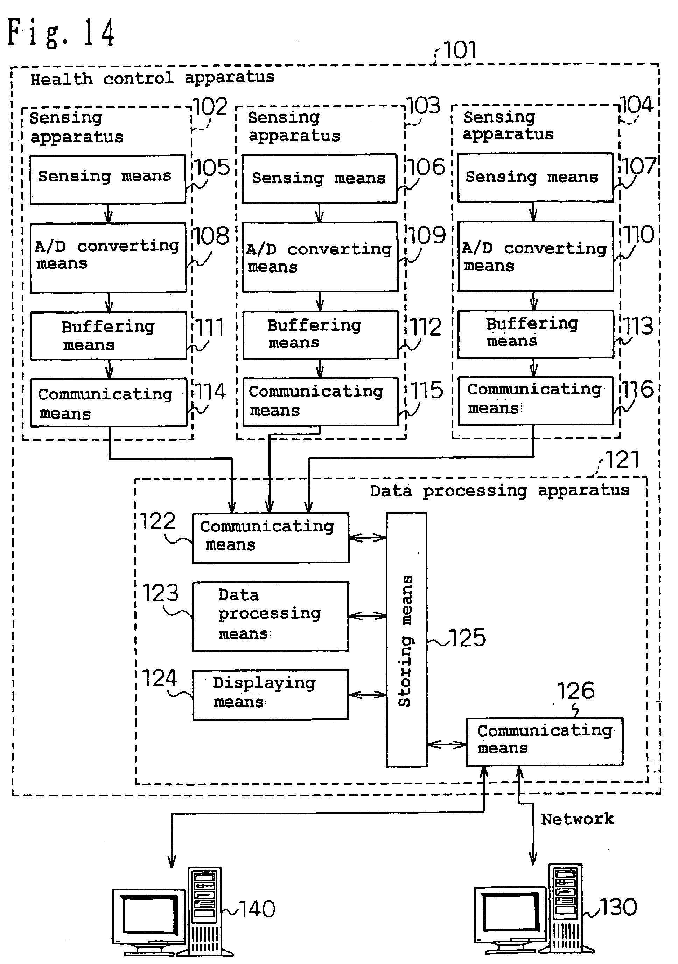

Figure 14 is a configuration diagram of a vital signs detection system according to Embodiment 4 of the invention. In the figure, like or corresponding parts to Figure 4 are designated by like numerals, and the description is omitted. An information server 130 is means of acquiring vital signs from the health control apparatus 101. A program server 140 is means of communicating with the data processing apparatus 121 of the health control apparatus 101 and thereby uploading a program and/or data to the data processing means 123 or the sensing means 105.

Described below is the operation of the vital signs detection system having the above-mentioned configuration according to Embodiment 4 of the invention. At the same time, described below is a vital signs detection method according to the invention.

In the present embodiment, the health control apparatus 101 operates similarly to Embodiments 1 and 2. In this embodiment, both or any of a program and data necessary for the operation of the sensing means 105-107 in the sensing apparatuses 102-104 are stored in the program server 140, whereby a program or the like necessary for each operation of the health control apparatus 101 is downloaded from the program server 140 to the storing means 125 of the data processing apparatus 121.

The communicating means 126 receives a command from the information server 130, whereby the data processing apparatus 121 and then the sensing apparatuses 102-104 are invoked. The data processing apparatus 121 acquires information on the types of the sensing apparatuses 102-104 via the communicating means 122, and then transmits the information through the communicating means 126 to the program server 140. In this embodiment, the sensing apparatuses 102-104 are composed of a consumption calorimeter, a perspirometer, and a tonometer, respectively.

On receiving the information on the type of each sensing means from the data processing apparatus 121, the program server 140 downloads a corresponding program and/or data into the health control apparatus 101.

On receiving the program and/or data, the health control apparatus 101 sets up the program and/or data into the sensing apparatuses 102-104. After the setup, the health control apparatus 101 operates similarly to Embodiment 1. At this time, an identical sensing apparatus may be used in different applications depending on the program and/or data. For example, the sensing apparatus 102 currently serving as a consumption calorimeter may be used as a tonometer when invoked next time.

As such, in the vital signs detection system according to the present embodiment, a program and/or data are downloaded from the program server 140 at each operation, whereby the health control apparatus 101 performs sensing operation on the basis of the program and/or data. This reduces the load onto the memory of the health control apparatus 101. Further, the operation is carried out on the basis of the latest program and/or data obtained from the program server 140. This permits a precise vital signs detection.

In this embodiment, the health control method according to Embodiment 3 maybe implemented using the vital signs detection system according to the invention. In this case, the information server 130 is managed by the instructor 302. Further, the information server 130 may perform also the function of the server 127 as described above.

In this embodiment, the data processing apparatus 121 of the health control apparatus 101 is an example of a terminal according to the present invention. Each sensing apparatus 102-104 is an example of detecting means according to the present invention. However, present invention is not restricted tothis. The entirety of the health control apparatus 101 including the sensing apparatuses 102-104 and the data processing apparatus 121 may be considered as an example of detecting means according to the present invention. Further, another terminal for communicating independently with the health control apparatus 101, the information server 130, and the program server 140 may be provided as a terminal according to the present invention. In this case, the programs and/or data of the data processing means 123 and the displaying means 124 in the data processing apparatus 121 can be stored in the program server 140. This reduces the load onto the health control apparatus 101. Further, distributable data within the health control program can be stored in the program server 140, and this reduces the load onto the information server 130.

(Embodiment 5)

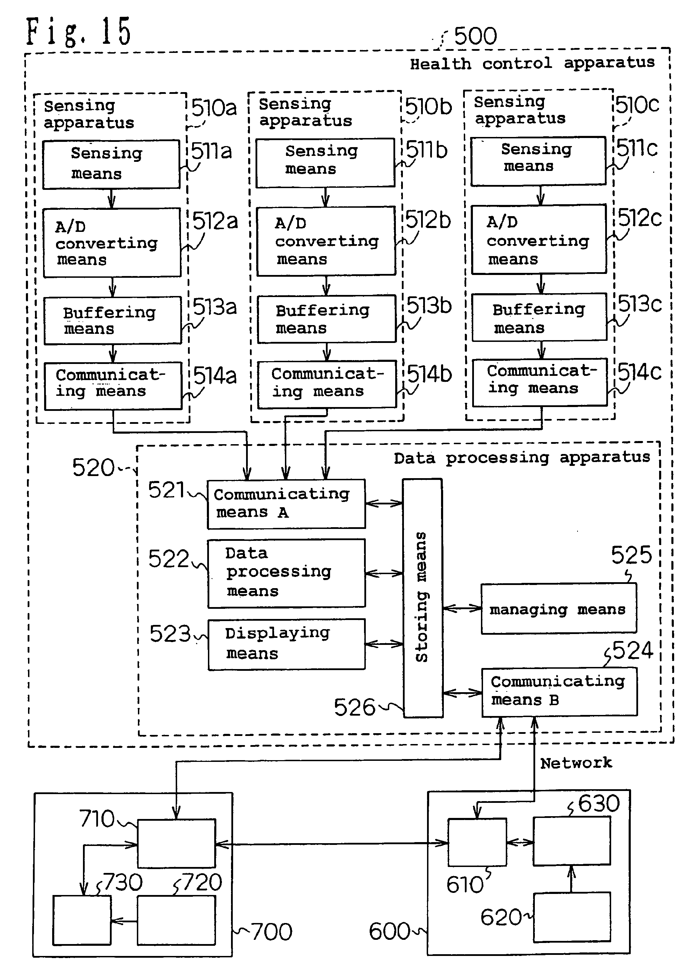

Figure 15 is a configuration diagram of a vital signs detection system according to Embodiment 5 of the invention. In the figure, the vital signs detection system comprises a health control apparatus 500, a program server 700, and an inspection item judgment server 600.

The health control apparatus 500 comprises one or more sensing apparatuses and a data processing apparatus. Illustrated in the figure is an exemplary health control apparatus comprising three sensing apparatuses 510a-510c and a data processing apparatus 520.

Each sensing apparatus 510a-510c comprises: each sensing means 511a-511c of detecting (sensing) vital signs from an organism; each A/D converting means 512a-512c of converting the detected vital signs into a digital signal; each buffering means 513a-513c of temporarily storing the vital data which is digital-converted vital signs; and each communicating means 514a-514c of communicating with the data processing apparatus 520 and transferring the detected vital data thereto.

The data processing apparatus 520 comprises: communicating means A 521 of communicating with the sensing apparatuses 510a-510c; data processing means 522 of processing the data of vital data and the like and thereby generating an inspection result; displaying means 523 of displaying the data of vital data and the like; storing means 526 of storing the data of vital data and the like; communicating means B 524 of communicating with the inspection item judgment server 600 and the program server 700 via a network; and managing means 525 of managing the operation of the entirety of the health control apparatus 500 including all the above-mentioned means and the sensing apparatuses 510a-510c.

The communicating means 514a-514c of the sensing apparatuses 510a-510c can communicate with the communicating means A 521 of the data processing apparatus 520. According to the specification, the communicating means 514a-514c cannot communicate with each other, or alternatively, these communicating means perform solely a relaying operation of transferring the data transmitted from another sensing apparatus connected to the same data processing apparatus, intact to the communicating means of another sensing apparatus or the data processing apparatus.

The data processing means 522 is composed of either dedicated hardware or a programmable device provided with a CPU, and thereby processes the data of vital signs and the like stored in the storing means 526. In case that the data processing apparatus 520 is composed of a programmable device, the function of the data processing means 522 is implemented by software which is stored in the storing means 526.

The managing means 525 manages the operation of all the above-mentioned means as well as the IDs each assigned to an organism of management target of the health control apparatus 500. The ID is used for identifying each target vital of the health control apparatus 500, and stored in the storing means 526.

The inspection item judgment server 600 comprises: communicating means 610 of obtaining information from the health control apparatus 500 via the network and communicating with the program server 700; a vitaldatabase 620 for storing information on the organisms of management target of the health control apparatus 500; and inspection item judgment server managing means 630 of processing the information obtained from the health control apparatus 500 and the information in the vital database 620 and thereby generating commands to the program server 700 and of managing the operation of the entirety of the inspection item judgment server 600 including the communicating means 610 and the vital database 620.

The program server 700 comprises: communicating means 710 of communicating with the inspection item judgment server 600 and the health control apparatus 500 and thereby exchanging the data; a program server main body 720 for storing the programs and data used for the operation of the sensing apparatuses 510a-510c and the data processing apparatus 520; and program server managing means 730 of managing the operation of the entirety of the program server 700 including the communicating means 710 and the program server main body 720.

In this embodiment, the health control apparatus 500 including the data processing apparatus 520 is an example of a terminal according to the present invention. Each sensing apparatus 510a-510c is an example of detecting means according to the present invention. The program server 700 is an example of a program server according to the present invention. The inspection item judgment server 600 is an example of an inspection item judgment server according to the present invention.

In the following description, the target vital of the health control apparatus 500 according to the present invention is a human being, that is, a person. Then, the health condition of the target person is controlled. In the vital database 620, information on the health condition of a human body including the information obtained by the health control apparatus 500 is stored in the form of a chart (referred to as "a target person chart," hereafter).

The storing means 526 in the data processing apparatus 520 of the health control apparatus 500 stores IDs (each referred to as "a target person ID, "hereafter) for identifying specific target persons. The target person IDs are managed by the managing means 525. Each target person ID is corresponded uniquely to each target person chart. In other words, the target person charts within the vital database 620 are classified and managed on the basis of the target person IDs.

In the following description, the sensing apparatus 510a is dedicated to detect the blood pressure of a target person. The sensing apparatus 510b is dedicated to detect the pulse wave of the target person. The sensing apparatus 510c is dedicated to detect the in-blood oxygen concentration of the target person.

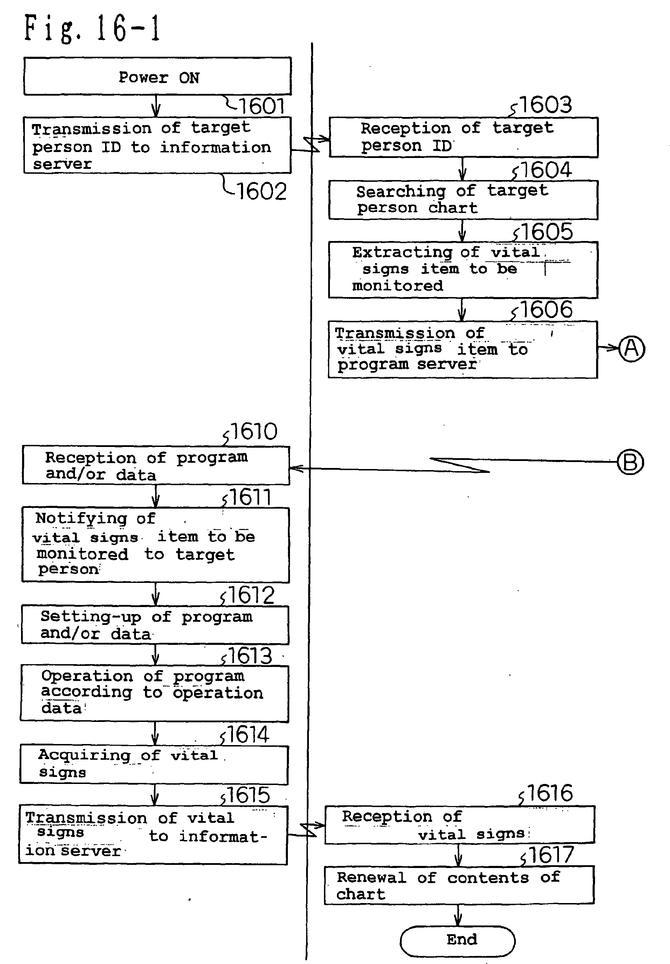

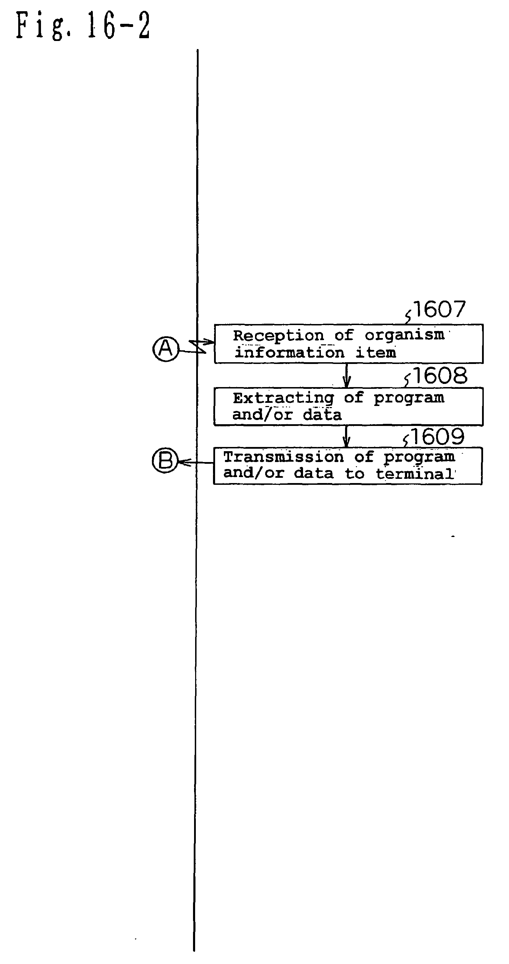

Described below is the operation of the vital signs detection system having the above-mentioned configuration according to Embodiment 5 of the invention. At the same time, a vital signs detection method according to the present invention and the operation of a terminal according to the present invention are described below with reference to the flowchart shown in Figure 16.

The health control apparatus 500 is powered ON and thereby begins to operate (Step 1601). In the data processing apparatus 520, the managing means 525 reads the target person ID from the storing means 526, and then the communicating means B 524 transmits the ID to the inspection item judgment server 600 via the network (Step 1602).

In the inspection item judgment server 600, the communicating means 610 receives the target person ID (Step 1603) . Then, the inspection item judgment server managing means 630 searches the vital database 620, and thereby selects a target person chart corresponding to the received target person ID (Step 1604) . Then, the inspection item judgment server managing means 630 determines whether any item needs inspection, or not, among the items of the target person's health condition in the target person chart. In case of necessity, the item is selected as an inspection item (Step 1605). For example, when the target person chart has an item "blood pressure," and when the blood pressure of the target person in the past one week falls outside a predetermined range of healthy condition, or alternatively when the value falls within the range but near the boundary, this item is set to be an inspection item.

The selected inspection item is transmitted from the communicating means B 524, through the network, to the program server 700 (Step 1606). At this time, the target person ID is transmitted together with the inspection item.

In the program server 700, the communicating means 710 receives the target person ID and the inspection item (Step 1607) . Then, the program server managing means 730 searches the program server main body 720, and thereby extracts a program or data corresponding to the inspection item (Step 1608). Then, the communicating means 710 transmits: the information containing the selected inspection item; and the extracted program; to the health control apparatus 500 (Step 1609). At this time, the address for transmission includes the previously received target person ID.

The health control apparatus 500 receives: the information containing the selected inspection item; and the extracted program; from the program server 700 (Step 1610). The managing means 525 refers to the target person ID received together with the program, and thereby checks whether the ID is the same as that previously transmitted to the inspection item judgment server 600 or not. When the received target person ID is different from that transmitted, the program is, for example, abandoned so as not to be used from now on in the health control apparatus 500.

When the received target person ID is the same as that transmitted, the managing means 525 displays the inspection item on the displaying means 523, thereby notifies the item to the target person (Step 1611).

Then, depending on the contents of the program, the managing means 525 sets up the program into the appropriate one among the sensing apparatuses 510a-510c and the data processing means 522 (Step 1612). For example, according to the above-mentioned example of inspection item, when the sensing apparatuses 510a-510c are in the initial state and not yet set up for detection operation, a program for causing the sensing apparatus 510a composed of a tonometer to operate is setup therein, while a program for causing the data processing means 522 to process the vital data from the sensing apparatus 510a as the blood pressure is set up therein.

Among the predetermined sensing apparatuses 510a-510c, any one into which a program is set up operates and acquires the vital data of the target person. The data is processed in the data processing apparatus 520, whereby vital signs is obtained (Step 1614). When the inspection item is the blood pressure, the sensing apparatus 510a serving as a tonometer measures the blood pressure of the target person. The result goes through the A/D converting means 512a, the buffering means 513a, the communicating means 514a, and is received by the communicating means A 521 of the data processing means 522, whereby the managing means 525 receives this blood pressure value as the vital sign.

The obtained vital signs is transmitted from the communicating means B 524, through the network, to the inspection item judgment server 600 (Step 1615). At the same time, the target person ID is transmitted.

The inspection item judgment server 600 receives the vital signs (Step 1616). Using the received vital signs, the inspection item judgment server 600 renews the contents of the target person chart corresponding to the target person ID within the vital database 620 (Step 1617), and completes the operation.

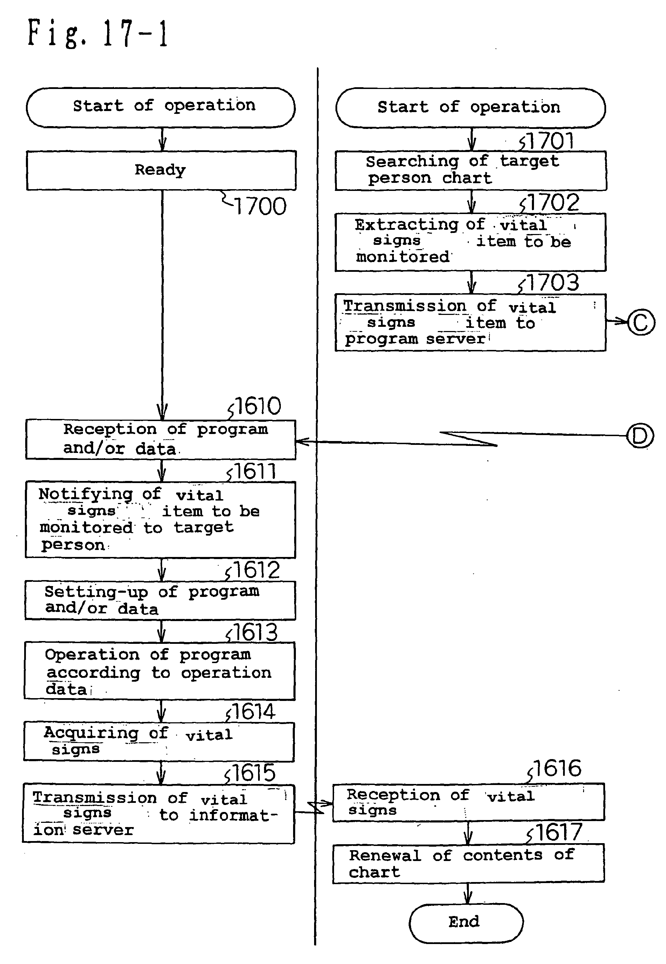

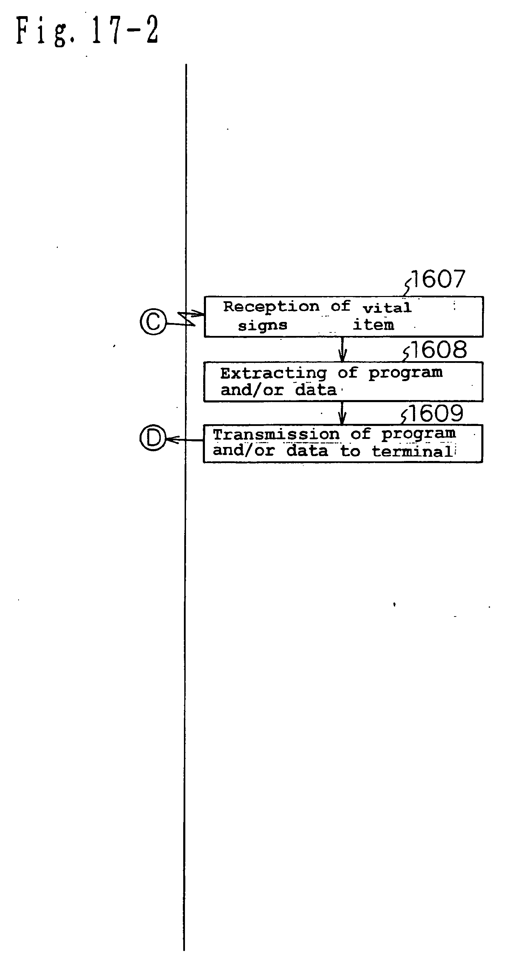

Another example of operation according to the present embodiment is described below with reference to the flowchart shown in Figure 17.

The health control apparatus 500 is powered ON and thereby begins to operate. In the data processing apparatus 520, an operation is taken so that the managing means 525 becomes ready and that the communicating means B 524 becomes ready for receiving data via the network (Step 1700).

When the inspection item judgment server 600 is powered ON and thereby begins to operate, the inspection item judgment server managing means 630 searches the vital database 620, and thereby selects a target person chart corresponding to a previously selected target person ID (Step 1701). The selecting of target person ID may be carried out by inputting from the outside at start-up of the inspection item judgment server 600. Alternatively, the target person ID may be that selected at previous operation. Further, the entirety of the vital database 620 may be searched, whereby vital signs which needs inspection, or an inspection item corresponding thereto, is extracted. Thus, the target person ID is obtained.

Then, the inspection item judgment server managing means 630 determines whether any item needs inspection, or not, among the items of the target person's health condition in the target person chart. In case of necessity, the item is selected as an inspection item (Step 1702). For example, when the target person chart has an item "blood pressure," and when the blood pressure of the target person in the past one year falls outside the range of healthy condition defined from the medical point of view, for example, on the basis of the average blood pressure of normal persons, or alternatively when the value falls within the range but near the boundary, this item is set to be an inspection item.

The selected inspection item is transmitted from the communicating means B 524, through the network, to the program server 700 (Step 1703). At this time, the target person ID is transmitted together with the inspection item.

Then, similarly to the exemplary operation shown in Figure 16, the program server 700 carries out the operation of Steps 1607-1609. The health control apparatus 500 carries out the operation of Steps 1610-1615. The inspection item judgment server 600 carries out the operation of Steps 1616-1617.

As such, in the vital signs detection system according to the present embodiment, at the start-up of the health control apparatus 500 or the inspection item judgment server 600, a program and/or data suitable for the inspection is downloaded from the program server 700 depending on the health condition of the organism of management target of the health control apparatus 500. On the basis of these program and/or data, the health control apparatus 500 performs sensing operation onto the organism. That is, an identical health control apparatus can perform a different type of inspection in each time of inspection. This avoids the necessity of preparing various inspection apparatuses in advance, and hence simplifies the inspection.

(Embodiment 6)

In the vital signs detection system according to the present embodiment, a health control apparatus continuously detects the vital signs of an organism. When a variation occurs in the detected data, the inspection item or the inspection method for the organism can be changed depending on the variation.

The configuration of the vital signs detection system according to the present embodiment is similar to that in Embodiment 5. Accordingly, description is carried out with reference to Figure 15, and detailed description is omitted.

Similarly to Embodiment 5, in the present embodiment, the health control apparatus 500 controls the health condition of a target person which is a target organism in the invention. In the vital database 620, information on the health condition of a human body including the information obtained by the health control apparatus 500 is stored in the form of a chart (referred to as "a target person chart," hereafter). Each target person chart is corresponded uniquely to each ID (referred to as "a target person ID," hereafter) which is stored in the storing means 526 in the data processing apparatus 520 of the health control apparatus 500 and is managed by the managing means 525. In other words, the target person charts within the vital database 620 are classified and managed on the basis of the target person IDs.

The sensing apparatus 510a is dedicated to detect the blood pressure of a target person. The sensing apparatus 510b is dedicated to detect the pulse wave of the target person. The sensing apparatus 510c is dedicated to detect the in-blood oxygen concentration of the target person.

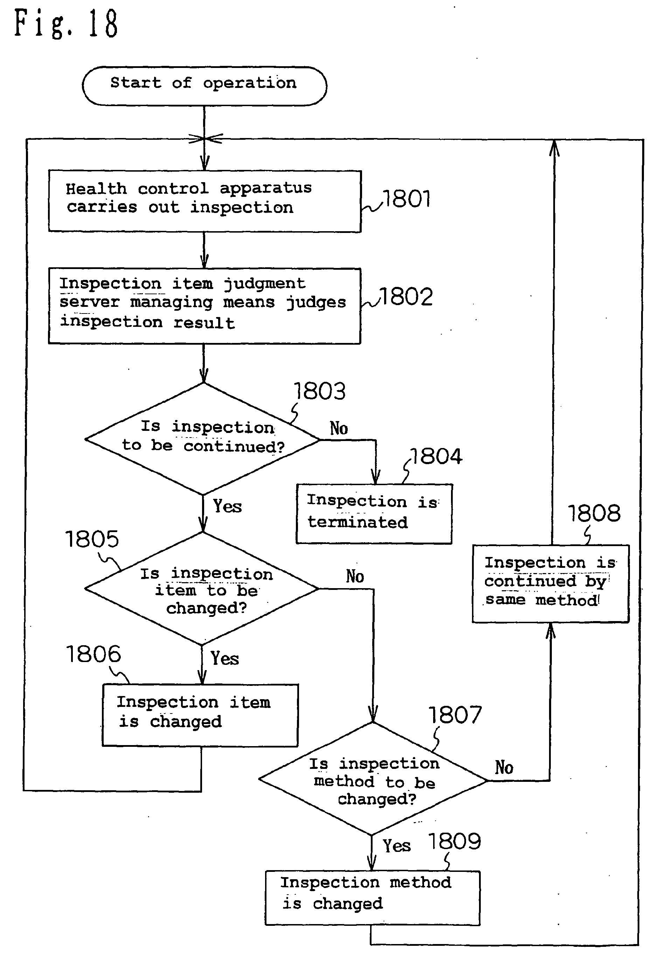

Described below is the operation of the vital signs detection system having the above-mentioned configuration according to Embodiment 6 of the present invention. At the same time, a vital signs detection method according.to the present invention and the operation of a terminal according to the present invention are described below with reference to the flowchart shown in Figure 18. As for the detailed operation of each means in the health control apparatus 500, the inspection item judgment server 600, and the program server 700, similar points to those in Embodiment 5 are omitted, and differences are solely described.

The health control apparatus 500 performs inspection to obtain the blood pressure value as vital signs, and then transmits the data to the inspection item judgment server 600 (Step 1801). The inspection item judgment server 600 receives the vital signs. Using the received vital signs, the inspection item judgment server 600 renews the contents of the target person chart corresponding to the target person ID within the vital database 620. At the same time, on the basis of the inspection result of the vital signs, the inspection item judgment server managing means 630 begins to determine how to proceed the subsequent operation (Step 1802).

In the determination, it is first determined whether the inspection is to be continued or not (Step 1803). When the inspection is determined not to be continued, the inspection item judgment server 600 notifies the termination of the operation to the health control apparatus 500. On receiving the notification, the health control apparatus 500 terminates the operation of the sensing apparatus 510a. On recognizing the termination of the operation of the health control apparatus 500, the inspection item judgment server 600 terminates the operation thereof.

In contrast, when the inspection is determined to be continued, the inspection item judgment server managing means 630 determines whether the inspection item is to be changed or not (Step 1804).

When the inspection item is to be changed, the inspection item judgment server 600 changes the inspection item, and then transmits the changed inspection item to the program server 700. On receiving the changed inspection item, the program server 700 extracts a program and/or data corresponding to the inspection item, and then transmits the program and/or data together with the information containing the changed inspection item, to the health control apparatus 500. The health control apparatus 500 receives the information containing changed inspection item and the program transmitted from the program server 700. Depending on the contents of the program, the health control apparatus 500 renews the program in a relevant apparatus or means among the sensing apparatuses 510a-510c and the data processing means 522 when another program is already set up. When no program is set up yet, the health control apparatus 500 sets up the new program (Step 1806).

On completion of the setup, the health control apparatus 500 begins to operate and perform inspection according to the new program, and then transmits newly obtained vital signs to the inspection item judgment server 600. That is, the entirety of the vital signs detection system repeats the operation of Step 1801 and the subsequent steps.

In contrast, when the inspection item is not to be changed, the inspection item judgment server managing means 630 determines that the acquisition of the vital signs of the same inspection item as previous is to be continued. Then, the inspection item judgment server managing means 630 determines whether the inspection method is to be changed into another inspection item or not (Step 1807).

When the inspection method is determined not to need a change, the inspection item judgment server 600 determines to continue the operation onto the same inspection item by the same inspection method as those determined in Step 1802, and then notifies the continuation of operation to the health control apparatus 500 (Step 1808). On receiving the notification, the health control apparatus 500 continues the operation of the sensing apparatus 510a, thereby begins inspection, and then transmits the newly obtained vital signs to the inspection item judgment server 600. The entirety of the vital signs detection system repeats the operation of Step 1801 and the subsequent steps.

In contrast, when the inspection method is to be changed, the inspection item judgment server 600 changes the inspection method, and then transmits the changed inspection method to the program server 700. On receiving the changed inspection method, the program server 700 extracts a program and/or data corresponding to the inspection method, and then transmits the program and/or data together with the information containing the changed inspection method, to the health control apparatus 500. The health control apparatus 500 receives the information containing changed inspection method and the program transmitted from the program server 700. Depending on the contents of the program, the health control apparatus 500 renews the program in a relevant apparatus or means among the sensing apparatuses 510a-510c and the data processing means 522 when another program is already set up. When no program is set up yet, the health control apparatus 500 sets up the new program (Step 1809).

On completion of the setup, the health control apparatus 500 begins to operate and perform inspection according to the new program, and then transmits newly obtained vital signs to the inspection item judgment server 600. That is, the entirety of the vital signs detection system repeats the operation of Step 1801 and the subsequent steps.

Described below are examples of the change of the inspection item in the above-mentioned Step 1806.

A first example of the change of the inspection item is as follows. At the start-up of the health control apparatus 500, the sensing apparatus 510a among the sensing apparatuses 510a-510c works as a waveform detector for detecting the waveform of the heart beat as a pulse wave, while the managing means 525 transmits the waveform itself as the vital signs to the inspection item judgment server 600.

Then, in an abnormal case in which the detected waveform has a pattern different from that of normal persons previously stored, the inspection item judgment server 600 provided with optional inspection items used for detecting the cause selects an option according to a predetermined order or on the basis of the other vital signs described in the target person chart.

When the heart rate is selected as a new inspection item and then transmitted to the program server 700, the program server 700 extracts a program for causing the data processing means 522 of the health control apparatus 500 to extract solely the peaks from the waveform detected by the sensing apparatus and thereby process the peaks into the heart rate, and thereby transmits the program to the health control apparatus 500.

The health control apparatus 500 sets up this program into the data processing means 522, and thereby changes the inspection item from the detection of the heart beat waveform into the detection of the heart rate. Then, the transmission of the vital signs is continued.

A second example of the change of the inspection item is described below. At the start-up of the health control apparatus 500, the sensing apparatus 510a among the sensing apparatuses 510a-510c works, and the vital signs detected by the health control apparatus 500 is the blood pressure value.

Then, for example, when the blood pressure value falls outside the range of normal persons, the inspection item judgment server 600 provided with optional inspection items used for detecting the cause selects an option according to a predetermined order or on the basis of the other vital signs described in the target person chart.

When the oxygen consumption by cardiac muscles, that is, so-called double product, is selected as a new inspection item and then transmitted to the program server 700, the program server 700 extracts a program and data for causing the data processing means 522 of the health control apparatus 500 to calculate the double product as the vital signs. The double product is the product of the heart rate and the blood pressure value. Accordingly, in addition to the program currently used for the detection of the blood pressure value by the sensing apparatus 510a, a program for causing another sensing apparatus to detect the heart rate and a program used for the calculation of the double product from the heart rate and the blood pressure value are extracted and transmitted to the health control apparatus 500.

The health control apparatus 500 sets up the program used for the detection of the heart rate, into the-sensing apparatus 510b, and then sets up the program used for the calculation of the double product, into the data processing means 522. Accordingly, in the health control apparatus 500, the sensing apparatus 510b also operates in addition to the sensing apparatus 510a. Further, the inspection item is changed from the detection of the blood pressure value into the detection of the double product. Then, the transmission of the vital signs is continued.

A third example of the change of the inspection item is described below. When an inspection result similar to that in the second example is obtained, and thereby the double product is to be detected, but when the health control apparatus 500 comprises only one sensing apparatus 510a for detecting solely the blood pressure value, the program server 700 extracts a program and data for causing the data processing means 522 of the health control apparatus 500 to calculate the double product as the vital signs, and then transmits the program and data to the health control apparatus 500. At the same time, the program server 700 transmits: information on a sensing apparatus necessary in addition to the sensing apparatus 510a; and an operation command for causing the sensing apparatus to serve as a part of the health control apparatus 500, for example, so as to be capable of communicating with the data processing apparatus 520; to the health control apparatus 500.

On receiving the program, the health control apparatus 500 stores the program temporarily into the storing means 526 in the data processing apparatus 520. On receiving the operation command, the displaying means 523 displays the command, and thereby notifies and requests the target person or an immediate operator of the health control apparatus 500 to prepare the sensing apparatus necessary in addition to the sensing apparatus 510a.

On recognizing the operation command on the displaying means, the target person or the operator additionally prepares a sensing apparatus capable of detecting the heart rate, and then sets up the apparatus so as to operate as a part of the health control apparatus 500.

After the set-up, the program necessary for the operation of the new sensing apparatus 510b which has previously been stored in the storing means 526 is set up into the sensing apparatus. Accordingly, in the health control apparatus 500, the sensing apparatus 510b for detecting the heart rate also operates in addition to the sensing apparatus 510a. Further, the inspection item is changed from the detection of the blood pressure value into the detection of the double product. Then, the transmission of the vital signs is continued.

In the first example of the change, description has been made for the case that the inspection item is changed using the same one sensing apparatus. However, a plurality of the same sensing apparatuses may be used.

In the second example of the change, description has been made for the case that the inspection item is changed by changing the number of utilized sensing apparatuses from a single sensing apparatus into two sensing apparatuses. However, the inspection item may be changed by changing from a plurality of sensing apparatuses into a single sensing apparatus, or alternatively, from a plurality into another plurality.

In the third example of the change, description has been made for the case that the inspection item is changed by changing the configuration of the health control apparatus from a health control apparatus comprising a single sensing apparatus into a health control apparatus comprising a plurality of sensing apparatuses. However, the inspection item may be changed by changing the configuration of the health control apparatus from a health control apparatus comprising an arbitrary number of sensing apparatus into a health control apparatus comprising another arbitrary number of sensing apparatuses.

Further, the inspection item may be changed by a combination of all or part of the first through the third example of the change.

Described below is an example of the change of the inspection method in the above-mentioned Step 1809.

At the start-up of the health control apparatus 500, the body temperature is measured as the vital signs, and transmitted as the inspection item to the inspection item judgment server 600. The sensing apparatus 510a among the sensing apparatuses 510a-510c measures the variation in the body temperature by detecting the variation in the electric current. The data processing means 522 recognizes the temperature of the target person when the variation is in equilibrium.

Then, in an abnormal case in which the body temperature exceeds a predetermined threshold value for the temperature of normal persons, the inspection item judgment server 600 provided with optional inspection items used for detecting the cause selects an option according to a predetermined order or on the basis of the other vital signs described in the target person chart. However, when there is no reason to use another optional inspection item, the inspection item judgment server changes the inspection method. Also in this case, the inspection item judgment server provided with optional inspection method used for detecting the cause selects an option according to a predetermined order or on the basis of the other vital signs described in the target person chart.

The variation in the body temperature has been detected as the variation in the electric current. However, in the new inspection method, the temperature of the target person is obtained on the basis of the change rate of the change. This inspection method is transmitted to the program server 700. Then, the program server 700 extracts a program for causing the data processing means 522 of the health control apparatus 500 to measure the body temperature on the basis of the above-mentioned time rate of the change in the electric current, and thereby transmits the program to the health control apparatus 500.

The health control apparatus 500 sets up this program into the data processing means 522, and thereby changes the processing method but maintains the same detection item as previous in the sensing apparatus 510a. Then, the transmission of the vital signs is continued.

In the above-mentioned example of the change of the inspection method, description has been made for the case that the operation and the configuration of the sensing apparatus are not changed, but that the program for the data processing means 522 is changed. However, similar to the examples of the change of the inspection item, the inspection method may be changed by changing the configuration, the number, and the program of the sensing apparatus or apparatuses, or alternatively, by changing the program of the data processing means.

The above-mentioned description has been made for the case that the program for causing each sensing apparatus to operate is dedicated to a specific application, and that the inspection item or the inspection method is changed by changing the setup of the data processing program in the data processing apparatus. However, the setup of the program of the sensing apparatus may be changed. For example, in the above-mentioned example, the sensing apparatus 510a has measured the blood pressure. However, depending on the data or setup downloaded from the program server 700, the sensing apparatus may measure another inspection item such as in-blood oxygen concentration and blood sugar level. That is, in the invention, as long as the inspection item and/or the inspection method are changeable, a program and/or data may be downloaded and set up into any of the sensing apparatus and the data processing apparatus.

The above-mentioned description has been made for the case that the inspection item is first changed, and that the inspection method is then changed. However, the inspection method may be first changed, and then the inspection item may be changed.

As such, in the vital signs detection system according to the present embodiment, during the inspection of a vital by the vital signs detection system, a program and/or data suitable for the inspection is downloaded from the program server 700 depending on the health condition of the organism of management target of the health control apparatus 500. On the basis of these program and/or data, the health control apparatus 500 performs sensing operation onto the organism. That is, an identical health control apparatus can perform a different type of inspection in each time of inspection. This permits automatic and continuous management of the condition of an organism.

In Embodiments 5 and 6, the organism of target person and the like may be a person, an animal such as a domestic animal and a pet, or alternatively, a plant such as a crop and a garden plant.

In Embodiments 5 and 6, the information in the vital database has been managed using the target person Ids, and the terminal of the present invention detects a specific vital signs of the present invention as a vital signs of the present invention. However, the authentication based on the ID may be omitted, whereby vital signs may be detected from a plurality of organisms and an identical health control apparatus may be used in the management of the plurality of organisms requesting similar information.

The displaying means 523 ID may be omitted.

In Embodiments 5 and 6, the health control method according to Embodiment 3 may be implemented using the vital signs detection system according to the invention. In this case, the inspection item judgment server 600 is managed by the instructor 302. Further, the inspection item judgment server 600 may perform also the function of the server 127 as described above.

The entirety of the health control apparatus 500 including the sensing apparatuses 510a-510c and the data processing apparatus 520 may be considered as an example of detecting means according to the invention. Further, another terminal for communicating independently with the health control apparatus 500, the inspection item judgment server 600, and the program server 700 may be provided as a terminal according to the present invention. In this case, the programs and/or data of the data processing means 522 and the displaying means 523 in the data processing apparatus 520 can be stored in the program server 700. This reduces the load of the storing means of the health control apparatus 500. Further, distributable data within the vital database can be stored in the program server 700, and this reduces the load onto the inspection item judgment server 600.

In Embodiments 1-3, each sensing apparatus 102-104 corresponds to vital signs detecting means according to the present invention. Each buffering means 111-113 corresponds to buffering means according to the present invention. Each communicating means 114-116 corresponds to first communicating means according to the present invention. The data processing apparatus 121 corresponds to vital signs processing means according to the present invention. The communicating means 122 corresponds to second communicating means according to the present invention. The communicating means 126 corresponds to third communicating means according to the present invention. The storing means 125 corresponds to storing means according to the present invention. The data processing means 123 corresponds to processing means according to the present invention. The displaying means 124 corresponds to displaying means according to the present invention.

The above-mentioned description has been made for the case of a health control apparatus and a vital signs detection system according to the present invention. However, each of a health control apparatus and a vital signs detection system according to the present invention may be a program which causes a computer to serve as all orpart of : said terminal capable of being connected to detecting means of detecting vital signs; said program server for storing a program and/or data for causing said detecting means to operate; and said information server for communicating with said terminal; in said vital signs detection system according to the invention; and which works in cooperation with the computer.

A terminal according to the present invention may be a program which makes a computer operate as all or part of said terminal capable of being connected to detecting means of detecting vital signs and which works in cooperation with the computer.

A program server according to the present invention may be a program which makes a computer operate as all or part of said program server for storing a program and/or data for causing said detecting means capable of being connected to detecting means of detecting vital signs to operate and which works in cooperation with the computer.

An information server according to the present invention may be a program which makes a computer operate as all or part of said information server for communicating with said terminal capable of being connected to detecting means of detecting vital signs and which works in cooperation with the computer.

The present invention may be a program which makes a computer operate as all or part of said processing means of said vital signs processing means of said vital signs processing apparatus according to the invention and which works in cooperation with the computer.

The present invention may be a program which causes a computer to serve as all or part of said first, second, third, and fourth steps in said health control method according to the present invention and which works in cooperation with the computer.

The present invention may be data structure used in all or part of said first, second, third, and fourth steps in said health control method according to the present invention, in cooperation with a computer.

The present invention may be a program which causes a computer to serve as all or part of: said terminal having detecting means of detecting, from an organism, vital signs corresponding to a predetermined inspection item;

said program server for storing a program and/or data for causing said detecting means to operate; and said inspection item judgment server for communicating with said terminal and thereby judging said inspection item on the basis of said vital signs; in said vital signs detection system according to the present invention; and which works in cooperation with the computer.

The present invention may be a computer-readable medium carrying a program causing a computer to serve as all or part of: said terminal capable of being connected to detecting means of detecting vital signs; said program server for storing a program and/or data for causing said detecting means to operate; and said information server for communicating with said terminal; in said vital signs detection system according to the present invention; wherein said read-out program works in cooperation with said computer.

The present invention may be a computer-readable medium carrying a program causing a computer to serve as all or part of: said terminal having detecting means of detecting, from an organism, vital signs corresponding to a predetermined inspection item; said program server for storing a program and/or data for causing said detecting means to operate; and said inspection item judgment server for communicating with said terminal and thereby judging said inspection item on the basis of said vital signs; in said vital signs detection system according to the present invention; wherein said read-out program works in cooperation with said computer.

Part of the means (or apparatuses, devices, circuits, sections, and the like) according the present invention and part of the steps (or processes, operations, effects, and the like) according the present invention indicate: a piece or pieces of means among a plurality of pieces of said means and a step or steps among a plurality of said steps; or alternatively, part of function in a piece of means and part of operation in a step, respectively.

Part of the apparatuses (or devices, circuits, sections, and the like) according the present invention indicates: an apparatus or apparatuses among a plurality of apparatuses; part of means (or devices, circuits, sections, and the like) in an apparatus; or alternatively , part of function in a piece of means.

The scope of the present invention includes also a computer-readable recording medium in which a program according to the present invention is recorded.

Amode of use of a program according to the present invention may be that the program is recorded in a computer-readable recording medium and works in cooperation with a computer.

A mode of use of a program according to the present invention may be that the program is transmitted through a transmitting medium, read out by a computer, and works in cooperation with the computer.

Data structure according to the present invention includes a database, a data format, a data table, a data list, and a data type.

The scope of recording media according to the present invention includes a ROM and the like, while the scope of transmitting media according to the present invention includes a transmitting medium such as the Internet, as well as light, radio waves, acoustic waves, and the like.

As described above, a configuration according to the present invention may be implemented by software or by hardware.

The embodiments of the present invention have the following effects.

According to an embodiment of the present invention, a health control apparatus can be constituted of one or more sensing apparatuses and a data processing apparatus. Even in case of a plurality of measurement items, the data processing apparatus can perform a common processing (data storing, data processing, communication with a server, and the like). Accordingly, the present invention is more efficient than a case that a plurality of measurement instruments are provided independently. Further, a single data processing apparatus can manage the vital signs. Accordingly, a processing relating to various data can be carried out easily.

The data processing apparatus and the sensing apparatus are separated from each other. Accordingly, the data processing apparatus can be designed into an appropriate size by selecting the displaying means and the inputting means depending on the condition of a user and the application. On the other hand, the sensing apparatus solely performs the acquisition and the transmission of vital signs. This simplifies downsizing and weight reduction.

In case that the processing means of a data processing apparatus is composed of a programmable device having a CPU core, a process program for implementing the function can be downloaded from a server. This permits flexible customization of the health control apparatus.

A data processing means of data processing apparatus comprising frequency processing means, heart rate managing means, and calorie consumption calculating means can serve as a consumption calorimeter. The consumption calorimeter having this configuration can be connected to two or more sensing apparatuses. Accordingly, even when data cannot be acquired by one sensing apparatus, the calorie consumption can be calculated from the data obtained from another sensing apparatuses. Alternatively, when abnormal data are abandoned among the pulse wave data from these two or more sensing apparatuses, the reliability in the calculated calorie consumption is improved.

During the execution of the health control program of a user, measurement data outside a predicted range can be detected, whereby a warning button is additionally displayed for a health control instructor. This permits the requesting of modification and/or change of the program. When an instructor instructs the health control of many users, measurement results having a problem can be automatically extracted and treated.

A user can actively carry out the control cycle of the health control program. That is, the user carries out the operation indicated by an upload button displayed together with the measurement data, and thereby uploads the measurement data to the server and downloads a health control program for the next cycle. The user can actively upload the execution result, renew the measurement data in the server, and download a new health control program.

This upload operation is a single action. This single action causes the upload of the measurement data and the renewal of the health control program. Accordingly, this does not disturb the habit of the user continuing the program.

An instructor can actively carry out the control cycle of the health control program. That is, when the time stamp of measurement data is before a predetermined date and time, display data containing an upload button is generated and displayed on the server. When the operation indicated by the upload button is carried out, the measurement data in the health control apparatus of the user is acquired, and then a new health control program is written. Thus, this is a health control method suitable for a case that the health control apparatus and the server are always connected to each other.

The upload operation is a single action. This single action causes the upload of the measurement data and the renewal of the health control program. Accordingly, even when an instructor instructs the health control of many users, the operation is minimized.

As described above, the present invention provides a vital signs detection system and a health control method in which vital signs can be obtained suitably to various purposes and various types of necessary data depending on the purpose.

**Claims**

1. A vital signs detection system comprising:

   a terminal (121, 500) connected to detecting means (102-104, 510a-c) for detecting vital signs, said detecting means (102-104, 510a-c) comprising an operation switch;
   a program server (140, 700) for storing a program and/or data to operate said detecting means (102-104, 510a-c); and
   an information server (130, 600) comprising means for communicating with said terminal (121, 500); wherein:

   said terminal (121, 500) comprising means (120, 524) for transmitting information including the type of said detecting means (102-104, 510a-c) to said program server, when said operation switch of said detecting means is turned ON;
   said program server (140, 700) comprising means for transmitting a predetermined program and/or data corresponding to said information to said terminal (121, 500) on receiving said information;
   said terminal (121, 500) comprising means for performing said predetermined program to operate said detecting means (102-104, 510a-c), after having received said predetermined program;
   said detecting means (102-104, 510a-c) operates according to said predetermined program and/or data received by said terminal, and thereby detects vital signs and sends the detected vital signs to said terminal (121, 500); and
   said terminal (121, 500) comprising means (120, 524) for transmitting the vital signs detected by said detecting means (102-104, 510a-c) to said information server (130, 600).

2. A vital signs detection system according to claim 1, wherein
   the detecting means (102-104, 510a-c) detect vital signs corresponding to an inspection item; and
   the information server is an inspection item judgment server (600) communicating with said terminal (121, 500) and thereby judging said inspection item according to said vital signs; wherein:

   when said terminal (121, 500) is started, said inspection item judgment server (600) judges a predetermined inspection item according to said vital signs corresponding to said predetermined inspection item; and according to the result of said judgment, said inspection item judgment server (600) makes said terminal (121, 500) download a predetermined program and/or data from said program server (140, 700), and thereby makes said detecting means (102-104, 510a-c) operate according to said predetermined program and/or data.

3. A vital signs detection system according to claim 2; wherein:

   when said detecting means (102-104, 510a-c) detects said vital signs corresponding to a predetermined inspection item, said terminal (121, 500) transmits said vital signs to said inspection item judgment server (600); on receiving said detected vital signs, said inspection item judgment server (600) judges said predetermined inspection item according to said vital signs, and according to the result of said judgment, determines whether the operation of said detecting means (102-104, 510a-c) is to be continued or not; and

when the result of said determination is the continuation of the operation of said detecting means (102-104, 510a-c), said inspection item judgment server (600) makes said detecting means (102-104, 510a-c) perform the same operation as previous, or alternatively, to download a predetermined program and/or data from said program server and thereby operate according to said predetermined program and/or data.

4. A vital signs detection system according to any of Claims 1 - 3 wherein: said detecting means (102-104,510a-c) is connected to said terminal (121,500) in an attachable and detachable manner; and in response to said download of said predetermined program and/or data, the type and/or the number of said detecting means (102-104,510a-c) connected to said terminal (121,500) is changed.

5. A vital signs detection system according to any of claims 2 - 4 wherein
said download of said predetermined program and/or data is carried out in order to change said inspection item to which said operation of said detecting means (102-104,510a-c) corresponds:

6. A vital signs detection system according to any of Claims 2 - 5 wherein said download of said predetermined program and/or data is carried out in order to change the inspection method for the same inspection item to which said operation of said detecting means (102-104,510a-c) corresponds.

7. A vital signs detection system according to any of claims 2 - 6 wherein:

said judgment by said inspection item judgment server (600) is carried out according to specific vital signs as said vital signs, which is in specific relevance to an organism or detection target of said detecting means (102-104, 510a-c);
said terminal (121, 500) is provided with an ID corresponding to said specific vital signs; and
said inspection item judgment server (600) refers to said ID of said terminal (121, 500), and thereby carries out said judgment according to said specific vital signs.

8. A vital signs detection system according to Claim 7 wherein said ID is transmitted from said terminal (121, 500) to said inspection item judgment server (600), whereby said inspection item judgment server (600) can refer to said ID.

9. A vital signs detection system according to Claim 7 or 8 wherein said inspection item judgment server (600) retains said ID in advance.

10. A vital signs detection system according to any of claims 1 - 9, wherein said terminal further comprising vital signs processing means (121, 520) for processing, storing, and displaying said vital signs detected by said vital signs detecting means (102-104, 510a-c); wherein:

said vital signs detecting means (102-104, 510a-c) comprises at least:

buffering means (111-113, 513a-c) for temporarily storing said detected vital signs; and
first communicating means (114-116, 514a-c) for communicating with said vital signs processing means (121, 520);

said vital signs processing means (121, 520) comprises at least:

second communicating means (122, 521) for communicating with said vital signs detecting means (102-104, 510a-c);
storing means (125, 526) for storing said vital signs;
processing means (123, 522) for processing said vital signs stored in said storing means (125, 526) according to a predetermined program and/or data; and
displaying means (124, 523) for displaying said vital signs stored in said storing means (125, 526) and/or output data of said processing means (123, 522).

11. A vital signs detection system according to claim 10, wherein:

said buffering means (111-113, 513a-c) and said storing means (125, 526) comprise a removable medium which can be detached; and

said removable medium is transferred between said vital signs detecting means (102-104, 510a-c) and said vital signs processing means (121, 520), whereby the data stored in said removable medium is transferred.

12. A vital signs detection system according to Claim 10 or 11, wherein:

said vital signs detecting means (102-104, 510a-c) is composed of a pulse wave sensor for measuring the pulse wave of a user (301); and

said processing means (123, 522) comprises: frequency processing means (141) for performing FFT (fast Fourier transformation) processing onto the frequency of said pulse wave; heart rate measuring means (142) for measuring heart rate from the output of said frequency processing means; and calorie consumption calculating means (143) for calculating calorie consumption from said heart rate.

13. A vital signs detection system according to Claim 12 wherein:

said vital signs processing means (121, 520) further comprises FFT processing means of performing FFT processing onto said heart rate;

according to the result of said FFT processing, it is determined whether said user is exercising or not; and

when it is determined that said user is not exercising, and when said heart rate exceeds a predetermined set value, said calorie consumption calculating means does not use said measured heart rate, but calculates calorie consumption according to said user's resting heart rate stored previously.

14. A vital signs detection system according to any of Claims 1 - 13, wherein said terminal (121, 500) further comprising inputting means (128) for permitting a user to input: personal data including one's name, age, and sex; health control indices including daily, weekly, monthly, and final target values for calorie consumption; and exercise indices including upper and lower limits for heart rate at exercise, and exercise time.

15. A vital signs detection system according to Claim 14 wherein said health control indices and said exercise indices are displayed on said displaying means (124,523).

16. A vital signs detection system according to Claim 14 or 15, wherein said terminal (121, 500) further comprising notifying means (129) for warning said user when said heart rate falls outside the range between said upper and lower limits for heart rate having been input through said inputting means (128).

17. A vital signs detection system according to any of Claims 10 - 16 wherein:

said processing means (123, 522) performs: the accumulation of said calorie consumption; the calculation of the difference from said target value; the calculation of the degree of achievement to said target value; and the calculation of the expected time of achieving said target value at the current pace of calorie consumption; and then stores these data in a region different from that of said vital signs data, within said storing means; and said displaying means (124,523) displays: the time series of the change in said heart rate and said calorie consumption; said accumulated value of calorie consumption; and said expected time of achieving said target value.

18. A health control method used in a vital signs detection system according to any of claims 1-17, including the following steps:

the step in which when said operation switch of said detecting means (102-104, 510a-c) is turned on, said terminal (121, 500) transmits information including the type of said detecting means (102-104, 510a-c) to said program server (140, 700);

the step in which on receiving said information, said program server (140, 700) transmits (1609) a predetermined program and/or data corresponding to said information to said terminal (121, 500);

the step (1609) in which said terminal (121, 500), after having received (1610) said predetermined program, performs said predetermined program to operate said detecting means (102-104, 510a-c);

the step in which said detecting means (102-104, 510a-c) operates (1613) according to said predetermined program and/or data received by said terminal (121, 500), and thereby detects (1614) vital signs;

the step in which said detecting means (102-104, 510a-c) sends the detected vital signs to said terminal (121, 500); and

the step in which said terminal (121, 500) transmits (1615) the vital signs data detected by said detecting means (102-104, 510a-c) to said information server (130, 600).

**19.** A health control method according to claim 18 comprising:

a first step in which a server (127, 140, 700) generates a health control program (303) including all or part of the exercise indices, the exercise menu, and the health control indices of a user (301) of said vital signs detection system according to a user chart (304) containing vital signs including the height, the weight, the body fat percentage, and the temperature of said user (301), and then transmits said program to said terminal (121, 500); a second step in which said terminal (121, 500) receives said health control program (303), and in which said user (301) uses said terminal (121, 500) in accordance with said health control program (303), and thereby acquires said user's vital signs; a third step in which a server (127, 140, 700) renews said health control program (303) according to said acquired measurement data (305); and a fourth step in which when said measurement data (305) falls outside the range of the values set in said health control program (303), said terminal (121, 500) transmits warning information for requesting attention to a server (127, 130, 600); and wherein:

on recognizing said warning information, a health control instructor (302) determines and alters said exercise indices and/or said exercise menu in said health control program (303) according to said warning information; a server (127, 140, 700) transmits said altered exercise indices and/or exercise menu to said terminal (121, 500); and when said terminal (121, 500) receives said altered exercise indices and/or exercise menu, said user takes exercise according to said exercise indices and/or exercise menu.

**20.** A health control method according to Claim 18 or 19 wherein:

said terminal (121, 500) transmits prompt information for requesting the renewal of said measurement data (305), to said user (301); on recognizing said prompt information, said user (301) determines and operates said terminal (121, 500) according to said prompt information; and said terminal (121, 500) acquires new measurement data, and then transmits them to a server (127, 130, 140, 600, 700).

**21.** A health control method according to any of Claims 18 - 20 wherein:

a server (127, 130, 600) transmits prompt information for requesting the renewal of said acquired measurement data (305), to said user (301); on recognizing said prompt information, said user (301) of said terminal (121, 500) determines and operates a server (127, 130, 600) according said prompt information; and on receiving new measurement data (305), a server (127, 140, 700) generates a new health control program (303) based on said data.

**22.** A health control method according to Claim 20 or 21 wherein said prompt information is output when said measurement data (305) is not renewed for a predetermined time or longer.

**23.** A health control method according to any of Claims 20 - 22 wherein said prompt information includes the method of operation of said terminal (121, 500) for said user (301) to renew said measurement data (305).

**24.** A health control method according to any of Claims 20 - 23 wherein said prompt information includes the operation method of a server (127, 130, 140, 600, 700) for said user (301) of said vital signs detection system.

**25.** A health control method according to any of Claims 20 - 24 wherein:

a server (127, 130, 140, 600, 700) further comprises a user ID (identifier) table (320) for storing user IDs for corresponding the user chart (304) of each user (301) to that user uniquely; and

said user ID is transmitted together with said health control program (303) to said terminal (101,500).

26. A health control method according to any of claims 18 - 25 comprising:

a step including a detecting step of detecting, from an organism, vital signs corresponding to an inspection item;
a program storing step of storing a program and/or data to make detecting step carry out; and
an inspection item judging step of judging said inspection item according to said vital signs detected by said detecting step; wherein:

when said detecting step is started, in said inspection item judging step, said predetermined inspection item is judged according to said vital signs corresponding to said predetermined inspection item; and according to the result of said judgment, a predetermined program and/or data stored in said program storing step is downloaded for said step including said detecting step; whereby in said detecting step, operation is carried out according to said predetermined program and/or data.

27. A health control method according to any of claims 18 - 26 comprising:

a step including a detecting step of detecting, from an organism, vital signs corresponding to a predetermined inspection item;
a program storing step of storing a program and/or data make said detecting step carry out; and
an inspection item judging step of judging said inspection item according to said vital signs detected by said detecting step; wherein:

when said vital signs corresponding to said predetermined inspection item is detected in said detecting step, said vital signs is transmitted, in said step including said detecting step, to said inspection item judging step for processing;
in said inspection item judging step, said predetermined inspection item is judged according to said detected vital signs, and according to the result of said judgment, it is determined whether said detecting step is to be continued or not; and
when the result of said determination is the continuation of said detecting step, the same operation as previous is carried out in said detecting step, or alternatively, a predetermined program and/or data stored in said program storing step is downloaded and thereby operation is carried out according to said predetermined program and/or data.

28. A computer program product loadable in a digital computer comprising instructions for performing the steps of a method according to any of claims 18 - 27.

29. A computer processable medium comprising a computer program product according to claim 28.

**Patentansprüche**

1. System zum Erfassen von Lebenszeichen, das umfasst:

ein Endgerät (121, 500), das mit einer Erfassungseinrichtung (102-104, 510a-c) zum Erfassen von Lebenszeichen verbunden ist, wobei die Erfassungseinrichtung (102-104, 510a-c) einen Betätigungsschalter umfasst;
einen Programm-Server (140, 700) zum Speichern eines Programms und/oder von Daten zum Betätigen der Erfassungseinrichtung (102-104, 510a-c); und
einen Information-Server (130, 600), der eine Einrichtung zum Kommunizieren mit dem Endgerät (121, 500) umfasst, wobei:

das Endgerät (121, 500) eine Einrichtung (120, 524) umfasst, die Informationen einschließlich des Typs der Erfassungseinrichtung (102-104, 510a-c) zu dem Programm-Server sendet, wenn der Betätigungsschalter der Erfassungseinrichtung AN geschaltet ist;
wobei der Programm-Server (140, 700) eine Einrichtung umfasst, die beim Empfangen der Informationen ein vorgegebenes Programm und/oder Daten, die den Informationen entsprechen, zu dem Endgerät (121, 500) sendet;
das Endgerät (121, 500) eine Einrichtung umfasst, die das vorgegebene Programm durchführt, um die Erfassungseinrichtung (102-104, 510a-c) zu betätigen, nachdem sie das vorgegebene Programm empfangen hat;

die Erfassungseinrichtung (102-104, 510a-c) entsprechend dem vorgegebenen Programm und/oder den Daten, die von dem Endgerät empfangen werden, arbeitet und so Lebenszeichen erfasst und die erfassten Lebenszeichen zu dem Endgerät (121, 500) sendet; und

das Endgerät (121, 500) eine Einrichtung (120, 524) umfasst, die die durch die Erfassungseinrichtung (102-104, 510a-c) erfassten Lebenszeichen zu dem Informations-Server (130, 600) sendet.

**2.** System zum Erfassen von Lebenszeichen nach Anspruch 1, wobei

die Erfassungseinrichtung (102-104, 510a-c) Lebenszeichen erfasst, die einem Untersuchungsobjekt entsprechen; und

der Informations-Server ein Untersuchungsobjekt-Beurteilungsserver (600) ist, der mit dem Endgerät (121, 500) kommuniziert und so das Untersuchungsobjekt entsprechend den Lebenszeichen beurteilt; wobei

wenn das Endgerät (121, 500) in Gang gesetzt wird, der Untersuchungsobjekt-Beurteilungs-Server (600) ein vorgegebenes Untersuchungsobjekt gemäß den Lebenszeichen beurteilt, die dem vorgegebenen Untersuchungsobjekt entsprechen, und der Untersuchungsobjekt-Beurteilungs-Server (600) entsprechend dem Ergebnis der Beurteilung das Endgerät (121, 500) veranlasst, ein vorgegebenes Programm und/oder Daten von dem Programm-Server (140, 700) herunterzuladen und so die Erfassungseinrichtungen (102-104, 510a-c) gemäß dem vorgegebenen Programm und/oder den Daten arbeiten lässt.

**3.** System zum Erfassen von Lebenszeichen nach Anspruch 2, wobei:

wenn die Erfassungseinrichtung (102-104, 510a-c) die Lebenszeichen erfasst, die einem vorgegebenen Untersuchungsobjekt entsprechen, das Endgerät (121, 500) die Lebenszeichen zu dem Untersuchungsobjekt-Beurteilungs-Server (600) sendet;

beim Empfangen der erfassten Lebenszeichen der Untersuchungsobjekt-Beurteilungsserver (600) das vorgegebene Untersuchungsobjekt gemäß den Lebenszeichen beurteilt und gemäß dem Ergebnis der Beurteilung feststellt, ob der Betrieb der Erfassungseinrichtung (102-104, 510a-c) fortzusetzen ist oder nicht; und

wenn das Ergebnis der Feststellung die Fortsetzung des Betriebes der Erfassungseinrichtung (102-104, 510a-c) ist, der Untersuchungsobjekt-Beurteilungs-Server (600) die Erfassungseinrichtung (102-104, 510a-c) veranlasst, den gleichen Betrieb wie zuvor durchzuführen oder als Alternative dazu ein vorgegebenes Programm und/oder Daten von dem Programm-Server herunterzuladen und so gemäß dem vorgegebenen Programm und/oder den Daten zu arbeiten.

**4.** System zum Erfassen von Lebenszeichen nach einem der Ansprüche 1-3, wobei

die Erfassungseinrichtung (102-104, 510a-c) mit dem Endgerät (121, 500) anbringbar und abnehmbar verbunden ist und in Reaktion auf das Herunterladen des vorgegebenen Programms und/oder der Daten der Typ und/oder die Anzahl der Erfassungseinrichtungen (102-104, 510a-c), die mit dem Endgerät (121, 500) verbunden sind, geändert wird/werden.

**5.** System zum Erfassen von Lebenszeichen nach einem der Ansprüche 2-4, wobei das Herunterladen des vorgegebenen Programms und/oder der Daten ausgeführt wird, um das Untersuchungsobjekt zu verändern, dem der Betrieb der Erfassungseinrichtung (102-104, 510a-c) entspricht.

**6.** System zum Erfassen von Lebenszeichen nach einem der Ansprüche 2-5, wobei das Herunterladen des vorgegebenen Programms und/oder der Daten ausgeführt wird, um das Untersuchungsverfahren für das gleiche Untersuchungsobjekt zu ändern, dem der Betrieb der Erfassungseinrichtungen (102-104, 510a-c) entspricht.

**7.** System zum Erfassen von Lebenszeichen nach einem der Ansprüche 2-6, wobei

die Beurteilung durch den Untersuchungsobjekt-Beurteilungsserver (600) gemäß spezifischer Lebenszeichen als den Lebenszeichen ausgeführt wird, die spezifische Relevanz für einen Organismus oder ein Erfassungsziel der Erfassungseinrichtung haben;

das Endgerät (121, 500) mit einer Kennung versehen ist, die den spezifischen Lebenszeichen entspricht; und

der Untersuchungsobjekt-Beurteilungs-Server (600) auf die Kennung des Endgerätes (121, 500) Bezug nimmt und so die Beurteilung gemäß der spezifischen Lebenszeichen ausführt.

**8.** System zum Erfassen von Lebenszeichen nach Anspruch 7, wobei die Kennung von dem Endgerät (121, 500) zu dem Untersuchungsobjekt-Beurteilungs-Server (600) gesendet wird, so dass der Untersuchungsobjekt-Beurteilungsserver (600) auf die Kennung Bezug nehmen kann.

9. System zum Erfassen von Lebenszeichen nach Anspruch 7 oder 8, wobei der Untersuchungsobjekt-Beurteilungs-Server (600) die Kennung im Voraus hält.

10. System zum Erfassen von Lebenszeichen nach einem der Ansprüche 1-9, wobei das Endgerät des Weiteren eine Lebenszeichen-Verarbeitungseinrichtung (121, 520) umfasst, die die durch die Lebenszeichen-Erfassungseinrichtung (102-104, 510a-c) erfassten Lebenszeichen verarbeitet, speichert und anzeigt, wobei:

die Lebenszeichen-Erfassungseinrichtung (102-104, 510a-c) wenigstens umfasst:

eine Puffereinrichtung (111-113, 513a-c) zum temporären Speichern der erfassten Lebenszeichen; und eine erste Kommunikationseinrichtung (114-116, 514a-c) zum Kommunizieren mit der Lebenszeichen-Verarbeitungseinrichtung (121, 520); wobei die Lebenszeichen-Verarbeitungseinrichtung (121, 520) wenigstens umfasst:

eine zweite Kommunikationseinrichtung (122, 521) zum Kommunizieren mit der Lebenszeichen-Erfassungseinrichtung (102-104, 510a-c); eine Speichereinrichtung (125, 526) zum Speichern der Lebenszeichen; eine Verarbeitungseinrichtung (123, 522) zum Verarbeiten der in der Speichereinrichtung (126, 526) gespeicherten Lebenszeichen gemäß einem vorgegebenen Programm und/oder Daten; und eine Anzeigeeinrichtung (124, 623) zum Anzeigen der in der Speichereinrichtung (125, 526) gespeicherten Lebenszeichen und/oder Ausgeben von Daten der Verarbeitungseinrichtung (123, 522).

11. System zum Erfassen von Lebenszeichen nach Anspruch 10, wobei:

die Puffereinrichtung (111-113, 513a-c) und die Speichereinrichtung (125, 526) ein entfernbares Medium umfassen, das abgenommen werden kann; und das entfernbare Medium zwischen der Lebenszeichen-Erfassungseinrichtung (102-104, 510a-c) und der Lebenszeichen-Verarbeitungseinrichtung (121, 520) überführt werden kann, so dass die in dem entnehmbaren Medium gespeicherten Daten überführt werden.

12. System zum Erfassen von Lebenszeichen nach Anspruch 10 oder 11, wobei die Lebenszeichen-Erfassungseinrichtung (102-104, 510a-c) aus einem Pulswellensensor zum Messen der Pulswelle eines Benutzers (301) besteht; und die Verarbeitungseinrichtung (123, 522) umfasst:

eine Frequenzverarbeitungseinrichtung (141) zum Durchführen von Verarbeitung der Frequenz der Pulswelle durch schnelle Fourier-Transformation; eine Herzraten-Messeinrichtung (142) zum Messen der Herzrate anhand des Ausgangs der Frequenzverarbeitungseinrichtung; und eine Kalorienverbrauch-Berechnungseinrichtung (143) zum Berechnen von Kalorienverbrauch anhand der Herzrate.

13. System zum Erfassen von Lebenszeichen nach Anspruch 12, wobei die Lebenszeichen-Verarbeitungseinrichtung (121, 520) des Weiteren eine Einrichtung zur Verarbeitung durch schnelle Fourier-Transformation umfasst, die Verarbeitung der Herzrate durch schnelle Fourier-Transformation durchführt; entsprechend dem Ergebnis der Verarbeitung durch schnelle Fourier-Transformation festgestellt wird, ob der Benutzer Sport treibt oder nicht; und wenn festgestellt wird, dass der Benutzer nicht Sport treibt, und wenn die Herzrate einen vorgegebenen Sollwert übersteigt, die Kalorienverbrauch-Berechnungseinrichtung die gemessene Herzrate nicht verwendet, sondern Kalorienverbrauch entsprechend der zuvor gespeicherten Ruhe-Herzrate des Benutzers berechnet.

14. System zum Erfassen von Lebenszeichen nach einem der Ansprüche 1-13, wobei das Endgerät (121, 500) des Weiteren eine Eingabeeinrichtung (128) umfasst, die es einem Benutzer gestattet, einzugeben:

persönliche Daten einschließlich des Namens, des Alters und des Geschlechts, Gesundheitsüberwachungs-Kennziffern, einschließlich des täglichen, wöchentlichen, monatlichen und abschließenden Zielwertes für Kalorienverbrauch; und
Trainings-Kennziffern einschließlich einer Ober- und einer Untergrenze der Herzrate beim Training, und Trainingszeit.

**15.** System zum Erfassen von Lebenszeichen nach Anspruch 14, wobei die Gesundheitsüberwachungs-Kennziffern und die Trainings-Kennziffern auf der Anzeigeeinrichtung (124, 523) angezeigt werden.

**16.** System zum Erfassen von Lebenszeichen nach Anspruch 14 oder 15, wobei das Endgerät (121, 500) des Weiteren eine Mitteilungseinrichtung (129) umfasst, die den Benutzer warnt, wenn die Herzrate außerhalb des Bereiches zwischen der Ober- und der Untergrenze für die Herzrate liegt, die über die Eingabeeinrichtung (128) eingegeben worden sind.

**17.** System zum Erfassen von Lebenszeichen nach einem der Ansprüche 10-16, wobei:

die Verarbeitungseinrichtung (123, 522) durchführt:

die Akkumulation des Kalorienverbrauchs;
die Berechnung der Differenz zu dem Zielwert;
die Berechnung des Grades der Erreichung des Zielwertes; und
die Berechnung der erwarteten Zeit der Erreichung des Zielwertes bei dem aktuellen Schrittmaß des Kalorienverbrauchs;
und dann diese Daten in einem anderen Bereich als dem der Lebenszeichen-Daten in der Speichereinrichtung speichert; und
die Anzeigeeinrichtung (124, 523) anzeigt:

die Zeitreihe der Änderung der Herzrate und des Kalorienverbrauchs;
den akkumulierten Wert des Kalorienverbrauchs; und
die erwartete Zeit der Erreichung des Zielwertes.

**18.** Gesundheitsüberwachungsverfahren, das in einem System zum Erfassen von Lebenszeichen nach einem der Ansprüche 1-17 eingesetzt wird, wobei es die folgenden Schritte einschließt:

den Schritt, in dem, wenn der Betätigungsschalter der Erfassungseinrichtung (102-104, 510a-c) angeschaltet wird, das Endgerät (121, 500) Informationen einschließlich des Typs der Erfassungseinrichtung (102-104, 510a-c) zu dem Programmserver (140, 700) sendet;
der Schritt, in dem beim Empfangen der Informationen der Programm-Server (140, 700) ein vorgegebenes Programm und/oder Daten, die den Informationen entsprechen, zu dem Endgerät (102-104, 510a-c) sendet (1609);
der Schritt (1609), in dem das Endgerät (121, 500), nachdem es das vorgegebene Programm empfangen hat (1610), das vorgegebene Programm durchführt um die Erfassungseinrichtung (102-104, 510a-c) zu betreiben;
der Schritt, in dem die Erfassungseinrichtung (102-104, 510a-c) gemäß dem vorgegebenen Programm und/oder Daten, die durch das Endgerät (121, 500) empfangen werden, arbeitet (1613) und so Lebenszeichen erfasst (1614);
der Schritt, in dem die Erfassungseinrichtung (102-104, 510a-c) die erfassten Lebenszeichen zu dem Endgerät (121, 500) leitet; und
der Schritt, in dem das Endgerät (121, 500) die durch die Erfassungseinrichtung (102-104, 510a-c) erfassten Lebenszeichen zu dem Informations-Server (130, 600) sendet (1615).

**19.** Gesundheitsüberwachungsverfahren nach Anspruch 18, das umfasst:

einen ersten Schritt, in dem ein Server (127, 140, 700) ein Gesundheitsüberwachungsprogramm (303) erzeugt, das alle oder einen Teil der Trainings-Kennziffern, das Trainings-Menü und die Gesundheitsüberwachungs-Kennziffern eines Benutzers (301) des Systems zum Erfassen von Lebenszeichen gemäß einer Benutzertabelle (304) erzeugt, die Lebenszeichen einschließlich der Körpergröße, des Gewichts, des Körper-Fettanteils und der Temperatur des Benutzers (301) enthält, und dann das Programm zu dem Endgerät (121, 500) sendet;
einen zweiten Schritt, in dem das Endgerät (121, 500) das Gesundheitsüberwachungsprogramm (303) empfängt und in dem der Benutzer (301) das Endgerät (121, 500) gemäß dem Gesundheitsüberwachungs-Programm (303) verwendet und so die Lebenszeichen des Benutzers erfasst;

einen dritten Schritt, in dem ein Server (127, 140, 700) das Gesundheitsüberwachungs-Programm (303) gemäß den erfassten Messdaten (305) erneuert; und

einen vierten Schritt, in dem, wenn die Messdaten (305) außerhalb des Bereiches der in dem Gesundheitsüberwachungs-Programm (303) eingestellten Werte liegen, das Endgerät (121, 500) Warninformationen, die Beachtung erfordern, zu einem Server (127, 130, 600) sendet; und wobei

beim Erkennen der Warninformationen eine Gesundheitsüberwachungs-Anweisungseinrichtung (302) die Trainings-Kennziffern und/oder das Trainings-Menü in dem Gesundheitsüberwachungs-Programm (303) gemäß den Warninformationen bestimmt und ändert;

ein Server (127, 140, 700) die geänderten Trainings-Kennziffern und/oder das Trainings-Menü zu dem Endgerät (121, 500) sendet; und

wenn das Endgerät (121, 500) die geänderten Trainings-Kennziffern und/oder das Trainings-Menü empfängt, der Benutzer entsprechend den Trainings-Kennziffern und/oder dem Trainings-Menü trainiert.

20. Gesundheitsüberwachungs-Verfahren nach Anspruch 18 oder 19, wobei:

das Endgerät (121, 500) Aufforderungsinformationen, die die Erneuerung der Messdaten (305) anfordern, zu dem Benutzer (301) sendet;

der Benutzer (301) beim Erkennen der Aufforderungsinformationen das Endgerät (121, 500) gemäß den Aufforderungsinformationen bestimmt und betätigt; und

das Endgerät (121, 500) neue Messdaten erfasst und sie dann zu einem Server (127, 130, 140, 600, 700) sendet.

21. Gesundheitsüberwachungsverfahren nach einem der Ansprüche 18-20, wobei:

ein Server (127, 130, 600) Aufforderungs-Informationen, die die Erneuerung der erfassten Messdaten (305) anfordern, zu dem Benutzer (301) sendet;

der Benutzer (301) des Endgerätes (121, 500) beim Erkennen der Aufforderungs-Informationen einen Server (127, 130, 600) gemäß den Aufforderungs-Informationen bestimmt und betätigt; und

ein Server (127, 140, 700) beim Empfangen neuer Messdaten (305) ein neues Gesundheitsüberwachungs-Programm (303) auf Basis der Daten erzeugt.

22. Gesundheitsüberwachungsverfahren nach Anspruch 20 oder 21, wobei die Aufforderungs-Informationen ausgegeben werden, wenn die Messdaten (305) über eine vorgegebene Zeit oder länger nicht erneuert werden.

23. Gesundheitsüberwachungs-Verfahren nach einem der Ansprüche 20-22, wobei die Aufforderungsinformationen das Verfahren des Betätigens des Endgerätes (121, 500) für den Benutzer (301) zum Erneuern der Messdaten (305) enthalten.

24. Gesundheitsüberwachungsverfahren nach einem der Ansprüche 20-23, wobei die Aufforderungs-Informationen das Verfahren zum Betätigen eines Servers (127, 130, 140, 600, 700) für den Benutzer (301) des Systems zum Erfassen von Lebenszeichen enthalten.

25. Gesundheitsüberwachungs-Verfahren nach einem der Ansprüche 20-24, wobei:

ein Server (127, 130, 140, 600, 700) des Weiteren eine Benutzerkennungs-Tabelle (320) zum Speichern von Benutzer-Kennungen umfasst, um eindeutige Entsprechungen der Benutzer-Tabelle (304) jedes Benutzers (301) zu diesem Benutzer herzustellen; und

die Benutzer-Kennung zusammen mit dem Gesundheitsüberwachungs-Programm (303) zu dem Endgerät (104) gesendet wird.

26. Gesundheitsüberwachungssystem nach einem der Ansprüche 18-25, das umfasst:

einen Schritt, der einen Erfassungsschritt des Erfassens von Lebenszeichen, die einem Untersuchungsobjekt entsprechen, von einem Organismus einschließt;

einen Programmspeicherschritt des Speicherns eines Programms und/oder von Daten, um Ausführen des Erfassungsschrittes zu veranlassen; und

einen Untersuchungsobjekt-Beurteilungsschritt des Beurteilens des Untersuchungsobjektes gemäß den mit dem Erfassungsschritt erfassten Lebenszeichen; wobei

wenn der Erfassungsschritt in Gang gesetzt wird, in dem Untersuchungsobjekt-Feststellschritt das vorgegebene Untersuchungsobjekt gemäß den Lebenszeichen beurteilt wird, die dem vorgegebenen Untersuchungsobjekt entsprechen, und gemäß dem Ergebnis der Beurteilung ein vorgegebenes Programm und/oder Daten, die in dem Programmspeicherschritt gespeichert werden, für den Schritt heruntergeladen werden, der den Erfassungsschritt einschließt, so dass in dem Erfassungsschritt Betrieb gemäß dem vorgegebenen Programm und/oder den Daten ausgeführt wird.

**27.** Gesundheitsüberwachungsverfahren nach einem der Ansprüche 18-26, das umfasst:

einen Schritt, der einen Erfassungsschritt des Erfassens von Lebenszeichen, die einem vorgegebenen Untersuchungsobjekt entsprechen, von einem Organismus einschließt;
einen Programmspeicherschritt des Speichern eines Programms und/oder von Daten, um Ausführung des Erfassungsschritts zu veranlassen; und
einen Untersuchungsobjekt-Beurteilungsschritt des Beurteilens des Untersuchungsobjektes gemäß dem mit dem Erfassungsschritt erfassten Lebenszeichen; wobei
wenn die Lebenszeichen, die dem vorgegebenen Untersuchungsobjekt entsprechen, in dem Erfassungsschritt erfasst werden, die Lebenszeichen in dem Schritt, der den Erfassungsschritt einschließt, zur Verarbeitung zu dem Untersuchungsobjekt-Beurteilungsschritt zur Verarbeitung gesendet werden;
in dem Untersuchungsobjekt-Beurteilungsschritt das vorgegebene Untersuchungsobjekt gemäß den erfassten Lebenszeichen beurteilt wird und gemäß dem Ergebnis der Beurteilung festgestellt wird, ob der Erfassungsschritt fortzusetzen ist oder nicht; und
wenn das Ergebnis der Feststellung die Fortsetzung des Erfassungsschrittes ist, der gleiche Betrieb wie zuvor in dem Erfassungsschritt ausgeführt wird, oder als Alternative dazu ein vorgegebenes Programm und/oder Daten, die in dem Programmspeicherschritt gespeichert werden, heruntergeladen werden und so Betrieb gemäß dem vorgegebenen Programm und/oder Daten ausgeführt wird.

**28.** Computerprogrammerzeugnis, das in einen digitalen Computer geladen werden kann und Befehle zum Durchführen der Schritte eines Verfahrens nach einem der Ansprüche 18-27 umfasst.

**29.** Durch Computer verarbeitbares Medium, das ein Computerprogrammerzeugnis nach Anspruch 28 umfasst.

**Revendications**

**1.** Système de détection de signes vitaux comprenant :

un terminal (121, 500) relié à un moyen de détection (102-104, 510a-c) destiné à détecter des signes vitaux, ledit moyen de détection (102-104, 510a-c) comprenant un interrupteur ;
un serveur de programme (140, 700) destiné à stocker un programme et/ou des données pour faire fonctionner ledit moyen de détection (102-104, 510a-c); et
un serveur d'information (130, 600) comprenant un moyen destiné à communiquer avec ledit terminal (121, 500); dans lequel :

ledit terminal (121, 500) comprend un moyen (120, 524) destiné à transmettre l'information incluant le type dudit moyen de détection (102-104, 510a-c) audit serveur de programme, lorsque ledit interrupteur du moyen de détection est en position « marche » ;
ledit serveur de programme (140, 700) comprend un moyen destiné à transmettre un programme et/ou des données prédéterminés correspondant à ladite information audit terminal (121, 500) en recevant ladite information ;
ledit terminal (121, 500) comprend un moyen destiné à exécuter ledit programme prédéterminé pour faire fonctionner ledit moyen de détection (102-104, 510a-c) après avoir reçu ledit programme prédéterminé ;
ledit moyen de détection (102-104, 510a-c) fonctionne selon lesdits programme et/ou données prédéterminés reçus par ledit terminal, et en conséquence détecte des signes vitaux et envoie les signes vitaux détectés audit terminal (121, 500); et
ledit terminal (121, 500) comprend un moyen destiné à transmettre (120, 524) les signes vitaux détectés par ledit moyen de détection (102-104, 510a-c) audit serveur d'information (130, 600).

**2.** Système de détection de signes vitaux selon la revendication 1, dans lequel

le moyen de détection (102-104, 510a-c) détecte des signes vitaux correspondant à un élément d'inspection ; et le serveur d'information est un serveur de jugement d'élément d'inspection (600) communiquant avec ledit terminal (121, 500) et en conséquence jugeant ledit élément d'inspection selon lesdits signes vitaux; dans lequel :

lorsque ledit terminal (121, 500) est démarré, ledit serveur de jugement d'élément d'inspection (600) juge un élément d'inspection prédéterminé selon lesdits signes vitaux correspondant audit élément d'inspection prédéterminé ; et en fonction du résultat dudit jugement, ledit serveur de jugement d'élément d'inspection (600) fait télécharger audit terminal (121, 500) un programme et/ou des données prédéterminés à partir dudit serveur de programme (140, 700) et en conséquence fait fonctionner ledit moyen de détection (102-104, 510a-c) selon lesdits programme et/ou données prédéterminés.

3. Système de détection de signes vitaux selon la revendication 2, dans lequel :

lorsque ledit moyen de détection (102-104, 510a-c) détecte lesdits signes vitaux correspondant à un élément d'inspection prédéterminé, ledit terminal (121, 500) transmet lesdits signes vitaux audit serveur de jugement d'élément d'inspection (600);
en recevant lesdits signes vitaux détectés, ledit serveur de jugement d'élément d'inspection (600) juge ledit élément d'inspection prédéterminé selon lesdits signes vitaux, et en fonction du résultat dudit jugement, détermine si l'opération dudit moyen de détection (102-104, 510a-c) doit être poursuivie ou non; et
lorsque le résultat de ladite détermination est la poursuite de l'opération dudit moyen de détection (102-104, 510a-c), ledit serveur de jugement d'élément d'inspection (600) fait exécuter audit moyen de détection (102-104, 510a-c) la même opération que précédemment, ou d'une autre façon, télécharger un programme et/ou des données prédéterminés à partir dudit serveur de programme, et en conséquence fonctionne selon lesdits programme et/ou données prédéterminés.

4. Système de détection de signes vitaux selon l'une quelconque des revendications 1 à 3, dans lequel: ledit moyen de détection (102-104, 510a-c) est relié audit terminal (121, 500) de manière amovible ; et en réponse audit téléchargement desdits programme et/ou données prédéterminés, le type et/ou le numéro dudit moyen de détection (102-104, 510a-c) relié audit terminal est modifié.

5. Système de détection de signes vitaux selon l'une quelconque des revendications 2 à 4, dans lequel ledit téléchargement desdits programme et/ou données prédéterminés est réalisé afin de modifier ledit élément d'inspection auquel ladite opération dudit moyen de détection (102-104, 510a-c) correspond.

6. Système de détection de signes vitaux selon l'une quelconque des revendications 2 à 5, dans lequel ledit téléchargement desdits programme et/ou données prédéterminés est réalisé afin de modifier le procédé d'inspection pour le même élément d'inspection auquel ladite opération dudit moyen de détection (102-104, 510a-c) correspond.

7. Système de détection de signes vitaux selon l'une quelconque des revendications 2 à 6, dans lequel:

ledit jugement par ledit serveur de jugement d'élément d'inspection (600) est réalisé selon des signes vitaux spécifiques comme lesdits signes vitaux, qui relèvent spécifiquement d'un organisme ou d'une cible de détection dudit moyen de détection (102-104, 510a-c);
ledit terminal (121, 500) est fourni avec un ID correspondant auxdits signes vitaux spécifiques ; et
ledit serveur de jugement d'élément d'inspection (600) se réfère audit ID dudit terminal (121, 500), et en conséquence réalise ledit jugement selon lesdits signes vitaux spécifiques.

8. Système de détection de signes vitaux selon la revendication 7, dans lequel ledit ID est transmis à partir dudit terminal (121, 500) audit serveur de jugement d'élément d'inspection (600) d'où il résulte que ledit serveur de jugement d'élément d'inspection (600) peut se référer audit ID.

9. Système de détection de signes vitaux selon la revendication 7 ou 8, dans lequel ledit serveur de jugement d'élément d'inspection (600) garde ledit ID par avance.

10. Système de détection de signes vitaux selon l'une quelconque des revendications 1 à 9, dans lequel ledit terminal comprend en outre un moyen de traitement des signes vitaux (121, 520) destiné à traiter, stocker et afficher lesdits signes vitaux détectés par ledit moyen de détection (102-104, 510a-c) de signes vitaux dans lequel :

ledit moyen de détection (102-104, 510a-c) de signes vitaux comprend au moins :

un moyen de tampon (111-113, 513a-c) pour stocker temporairement lesdits signes vitaux détectés ; et
un premier moyen de communication (114-116, 514a-c) destiné à communiquer avec ledit moyen de traitement (121, 520) de signes vitaux ;
ledit moyen de traitement (121, 520) de signes vitaux comprend au moins :

un second moyen de communication (122, 521) destiné à communiquer avec ledit moyen de détection (102-104, 510a-c) de signes vitaux ;
un moyen de stockage (125, 526) destiné à stocker lesdits signes vitaux ;
un moyen de traitement (123, 522) destiné à traiter lesdits signes vitaux stockés dans ledit moyen de stockage (125, 526) selon un programme et/ou des données prédéterminés; et
un moyen d'affichage (124, 523) destiné à afficher lesdits signes vitaux stockés dans ledit moyen de stockage (125, 526) et/ou des données de sortie dudit moyen de traitement (123, 522).

**11.** Système de détection de signes vitaux selon la revendication 10, dans lequel :

ledit moyen de tampon (111-113, 513a-c) et ledit moyen de stockage (125, 526) comprennent un support amovible pouvant être détaché ; et
ledit support amovible est transféré entre ledit moyen de détection (102-104, 510a-c) de signes vitaux et ledit moyen de traitement (121, 520) de signes vitaux, d'où il résulte que les données stockées dans ledit milieu amovible sont transférées.

**12.** Système de détection de signes vitaux selon la revendication 10 ou 11, dans lequel :

ledit moyen de détection (102-104, 510a-c) de signes vitaux est composé d'un détecteur d'onde pulsée destiné à mesurer l'onde pulsée d'un utilisateur (301); et
ledit moyen de traitement (123, 522) comprend : un moyen de traitement de la fréquence (141) destiné à réaliser le traitement de la TFR (transformation de Fourier rapide) sur la fréquence de ladite onde pulsée; un moyen de mesure de la fréquence cardiaque (142) destiné à mesurer la fréquence cardiaque à partir de la sortie dudit moyen de traitement de la fréquence ; et un moyen de calcul de la consommation calorique (143) destiné à calculer la consommation calorique à partir de ladite fréquence cardiaque.

**13.** Système de détection de signes vitaux selon la revendication 12, dans lequel :

ledit moyen de traitement (121, 520) de signes vitaux comprend en outre un moyen de traitement de la TFR destiné à réaliser le traitement de la TFR sur ladite fréquence cardiaque ;
en fonction du résultat dudit traitement de la TFR, il est déterminé si ledit utilisateur s'exerce ou non ; et
lorsqu'il est déterminé que ledit utilisateur ne s'exerce pas, et lorsque ladite fréquence cardiaque dépasse une valeur établie prédéterminée, ledit moyen de calcul de la consommation calorique n'utilise pas ladite fréquence cardiaque mesurée, mais calcule la consommation calorique selon la fréquence cardiaque au repos d'un utilisateur préalablement stockée.

**14.** Système de détection de signes vitaux selon l'une quelconque des revendications 1 à 13, dans lequel ledit terminal (121, 500) comprend en outre un moyen de saisie (128) destiné à permettre à un utilisateur de saisir : des données personnelles incluant le nom, l'âge et le sexe d'une personne ; des indices de contrôle de santé incluant les valeurs quotidiennes hebdomadaires et mensuelles et les valeurs cibles finales de la consommation calorique ; et des indices d'exercice incluant des limites supérieures et inférieures pour une fréquence cardiaque à l'exercice, et une durée d'exercice.

**15.** Système de détection de signes vitaux selon la revendication 14 dans lequel lesdits indices de contrôle de santé et lesdits indices d'exercice sont affichés sur ledit moyen d'affichage (124, 523).

**16.** Système de détection de signes vitaux selon la revendication 14 ou 15, dans lequel ledit terminal (121, 500) comprend en outre un moyen de notification (129) pour alerter ledit utilisateur lorsque ladite fréquence cardiaque tombe hors de la fourchette desdites limites supérieure et inférieure pour la fréquence cardiaque qui a été entrée via ledit moyen de saisie (128).

**17.** Système de détection de signes vitaux selon l'une quelconque des revendications 10 à 16, dans lequel :

ledit moyen de traitement (123, 522) réalise : l'accumulation de ladite consommation calorique ; le calcul de la différence par rapport à la valeur cible ; le calcul du degré de réalisation de ladite valeur cible ; et le calcul du temps attendu pour atteindre ladite valeur cible au rythme actuel de la consommation calorique ; puis stocke ces données dans une région différente de celle desdites données des signes vitaux, à l'intérieur dudit moyen de stockage ; et

ledit moyen d'affichage (124, 523) affiche : la série de temps de changement dans ladite fréquence cardiaque et ladite consommation calorique ; ladite valeur accumulée de la consommation calorique ; et ledit temps attendu pour atteindre ladite valeur cible.

**18.** Procédé de contrôle de santé utilisé dans un système de détection de signes vitaux selon l'une quelconque des revendications 1 à 17, incluant les étapes suivantes :

l'étape dans laquelle lorsque ledit interrupteur dudit moyen de détection (102-104, 510a-c) est sur la position « marche », ledit terminal (121, 500) transmet une information incluant le type dudit moyen de détection (102-104, 510a-c) audit serveur de programme (140-700);

l'étape dans laquelle en recevant ladite information, ledit serveur de programme (140, 700) transmet (1609) un programme et/ou des données prédéterminés correspondant à ladite information audit terminal (121, 500);

l'étape (1609) dans laquelle ledit terminal (121, 500), après avoir reçu (1610) ledit programme prédéterminé, exécute ledit programme prédéterminé pour faire fonctionner ledit moyen de détection (102-104, 510a-c).

l'étape dans laquelle ledit moyen de détection (102-104, 510a-c) fonctionne (1613) selon lesdits programme et/ou données prédéterminés reçus par ledit terminal (121, 500), et détecte (1614) en conséquence des signes vitaux ;

l'étape dans laquelle ledit moyen de détection (102-104, 510a-c) envoie les signes vitaux détectés audit terminal (121, 500) ; et

l'étape dans laquelle ledit terminal (121, 500) transmet (1615) les données des signes vitaux détectés par ledit moyen de détection (102-104, 510a-c) audit serveur d'information (130, 600).

**19.** Procédé de contrôle de santé selon la revendication 18 comprenant :

une première étape dans laquelle un serveur (127, 140, 700) génère un programme de contrôle de santé (303) incluant la totalité ou une partie des indices d'exercice, du menu d'exercice, et des indices de contrôle de santé d'un utilisateur (301) dudit système de détection de signes vitaux selon un diagramme d'utilisateur (304) contenant des signes vitaux incluant la taille, le poids, le pourcentage de graisse corporelle, et la température dudit utilisateur (301), et ensuite transmet ledit programme audit terminal (121, 500);

une seconde étape dans laquelle ledit terminal (121, 500) reçoit ledit programme de contrôle de santé (303), et dans laquelle ledit utilisateur (301) utilise ledit terminal (121, 500) selon ledit programme de contrôle de santé (303), et en conséquence acquiert lesdits signes vitaux de l'utilisateur ;

une troisième étape dans laquelle un serveur (127, 140, 700) renouvelle ledit programme de contrôle de santé (303) selon lesdites données de mesure acquises (305) ; et

une quatrième étape dans laquelle lorsque lesdites données de mesure (305) tombent hors de la fourchette des valeurs établies dans ledit programme de contrôle de santé (303), ledit terminal (121, 500) transmet une information d'alerte pour demander l'attention d'un serveur (127, 130, 600) ; et dans lequel :

en reconnaissant ladite information d'alerte, un instructeur de contrôle de santé (302) détermine et modifie lesdits indices d'exercice et/ou ledit menu d'exercice dans ledit programme de contrôle de santé (303) selon ladite information d'alerte ;

un serveur (127, 140, 700) transmet lesdits indices d'exercice et/ou menu d'exercice modifiés audit terminal (121, 500); et

lorsque ledit terminal (121, 500) reçoit lesdits indices d'exercice et/ou menu d'exercice modifiés, ledit utilisateur prend l'exercice selon lesdits indices d'exercice et/ou menu d'exercice.

**20.** Procédé de contrôle de santé selon la revendication 18 ou 19 dans lequel :

ledit terminal (121, 500) transmet une invite de commande destinée à demander le renouvellement desdites données de mesure (305) audit utilisateur (301);

en reconnaissant ladite invite de commande, ledit utilisateur (301) détermine et fait fonctionner ledit terminal (121, 500) selon ladite invite de commande; et

ledit terminal (121, 500) acquiert de nouvelles données de mesure, et ensuite les transmet à un serveur (127, 130, 140, 600, 700).

**21.** Procédé de contrôle de santé selon l'une quelconque des revendications 18 à 20 dans lequel :

un serveur (127, 130, 600) transmet une invite de commande destinée à demander le renouvellement desdites données de mesure acquises (305), audit utilisateur (301);

en reconnaissant ladite invite de commande, ledit utilisateur (301) dudit terminal (121, 500) détermine et fait fonctionner un serveur (127, 130, 600) selon ladite invite de commande ; et

en recevant les nouvelles données de mesure (305), un serveur (127, 140, 700) génère un nouveau programme de contrôle de santé (303) basé sur lesdites données.

**22.** Procédé de contrôle de santé selon la revendication 20 ou 21 dans lequel ladite invite de commande est sortie lorsque ladite donnée de mesure (305) n'est pas renouvelée pour un temps prédéterminé ou pour plus longtemps.

**23.** Procédé de contrôle de santé selon l'une quelconque des revendications 20 à 22 dans lequel ladite invite de commande inclut le procédé de fonctionnement dudit terminal (121, 500) pour ledit utilisateur (301) pour renouveler lesdites données de mesure (305).

**24.** Procédé de contrôle de santé selon l'une quelconque des revendications 20 à 23 dans lequel ladite invite de commande inclut le procédé de fonctionnement d'un serveur (127, 130, 140, 600, 700) pour ledit utilisateur (301) dudit système de détection de signes vitaux.

**25.** Procédé de contrôle de santé selon l'une quelconque des revendications 20 à 24 dans lequel :

un serveur (127, 130, 140, 600, 700) comprend en outre une table ID (identifiant) d'utilisateur (320) destiné à stocker des ID d'utilisateur pour faire correspondre le diagramme d'utilisateur (304) de chaque utilisateur (301) à cet utilisateur uniquement ; et

ledit ID d'utilisateur est transmis avec ledit programme de contrôle de santé (303) audit terminal (121, 500).

**26.** Procédé de contrôle de santé selon l'une quelconque des revendications 18 à 25 à comprenant :

une étape incluant une étape de détection destinée à détecter, à partir d'un organisme, des signes vitaux correspondant à un élément d'inspection ;

une étape de stockage de programme destinée à stocker un programme et/ou des données afin de réaliser l'étape de détection ; et

une étape de jugement de l'élément d'inspection destinée à juger ledit élément d'inspection selon lesdits signes vitaux détectés par ladite étape de détection ; dans lequel :

lorsque ladite étape de détection est démarrée, dans ladite étape de jugement de l'élément d'inspection, ledit élément d'inspection prédéterminé est jugé selon lesdits signes vitaux correspondant audit élément d'inspection prédéterminé ; et en fonction du résultat dudit jugement, un programme et/ou des données prédéterminés stockés dans ladite étape de stockage programme est téléchargé pour ladite étape incluant ladite étape de détection ; d'où il résulte que dans ladite étape de détection, l'opération est réalisée selon lesdits programme et/ou données prédéterminés.

**27.** Procédé de contrôle de santé selon l'une quelconque des revendications 18 à 26 comprenant :

une étape incluant une étape de détection destinée à détecter, à partir d'un organisme, des signes vitaux correspondant à un élément d'inspection prédéterminé ;

une étape de stockage de programme destinée à stocker un programme et/ou des données afin de réaliser l'étape de détection ; et

une étape de jugement de l'élément d'inspection pour juger ledit élément d'inspection selon lesdits signes vitaux détectés par ladite étape de détection ; dans lequel :

lorsque lesdits signes vitaux correspondant audit élément d'inspection prédéterminé sont détectés dans ladite étape de détection, lesdits signes vitaux sont transmis, dans ladite étape incluant ladite étape de détection, à ladite étape de jugement de l'élément d'inspection, pour traitement ;

dans ladite étape de jugement de l'élément d'inspection, ledit élément d'inspection prédéterminé est jugé selon lesdits signes vitaux détectés, et en fonction du résultat dudit jugement, il est déterminé si ladite étape de détection est poursuivie ou non ; et

lorsque le résultat de ladite détermination est la poursuite de ladite étape de détection, la même opération que précédemment est réalisée dans ladite étape de détection, ou d'une autre façon, un programme et/ou des données prédéterminés stockés dans ladite étape de stockage de programme est téléchargé et en conséquence l'opération est réalisée selon lesdits programme et/ou données prédéterminés.

**28.** Produit de programme informatique chargeable sur un ordinateur numérique comprenant des instructions d'exécution des étapes d'un procédé selon l'une quelconque des revendications 18 à 27.

**29.** Support informatique traitable comprenant un produit de programme informatique selon la revendication 28.

# Fig. 1

# Fig. 2

401 — Vital data

401a

401b

402 — Programs

400

403 — Parameters / initial setting data

# Fig. 3

S201 —— | Sensing of vital signs |

S202 —— | A/D conversion of data |

S203 —— | Storing of vital data temporarily in time series |

S204 —— | Transmission |

S205 —— | Reception |

S206 —— | Storing in storing means |

S207 —— | Processing of data |

S208 —— | Displaying of processing result |

# Fig. 4

# Fig. 5

Health control apparatus (consumption calorimeter) ␣101

Sensing apparatus ␣102

| Sensing means | 105 |

↓

| A/D converting means | 108 |

↓

| Buffering means | 111 |

↓

| Communicating means | 114 |

Data processing apparatus ␣121

122 | Communicating means | ↔

123 Data processing means

141 | Frequency processing means |

142 | Heart rate measuring means | ↔ Storing means 125

143 | Calorie consumption calculating means |

124 | Displaying means | ↔

# Fig. 6

# Fig. 7

```
┌─────────────────────────────────────────────────────────┐  ┌101
│           Health control apparatus (consumption calorimeter)│
│    ┌ ─ ─ ─ ─ ─ ─ ─ ─ ─ ─ ─ ─ ─ ─ ─ ─ ─ ─ ─ ─ ─ ┐        │
│       Sensing apparatus                                   │
│    │  ┌─────────────────────┐                    │        │
│       │   Sensing means     │      105                    │
│    │  └─────────────────────┘                    │        │
│  102            │                                          │
│    │            ▼                                 │        │
│       ┌─────────────────────┐                             │
│    │  │ A/D converting      │      108            │        │
│       │ means               │                             │
│    │  └─────────────────────┘                    │        │
│                 │                                          │
│    │            ▼                                 │        │
│       ┌─────────────────────┐                             │
│    │  │  Buffering means    │      111            │        │
│       └─────────────────────┘                             │
│    │            │                                 │        │
│                 ▼                                          │
│    │  ┌─────────────────────┐                    │        │
│       │  Communicating      │      114                    │
│    │  │  means              │                    │        │
│       └─────────────────────┘                             │
│    └ ─ ─ ─ ─ ─ ─│─ ─ ─ ─ ─ ─ ─ ─ ─ ─ ─ ─ ─ ─ ─ ─ ┘        │
```

Fig. 8

124 — Displaying means

125 — Storing means

Calorie consumption
Accumulated value
Remained calorie consumption
Time of achievement

Data

Heart rate
Calorie consumption

Calorie consumption, accumulated value
Accumulated value

Accumulated value and target value
Remained calorie consumption

Calorie consumption
Remained calorie consumption
Time of achievement

143 — Calorie consumption calculating means

151 — Calculation section

152 — Accumulation section

153 — Subtraction section

154 — Achievement time calculation section

# Fig. 9

127

**Server**

306

Warning button

Modification and /or change

303

Health control program

305

Measurement data

304

User chart

302

Exercise menu

Exercise indices

Health control indices

Measure-ment data

101

**Health control apparatus**

Storing means

Communicating means

125

122

301

Displaying means

124

Inputting means

128

# Fig. 10

```
      ┌──────────┐
      │  Start   │
      └──────────┘
           │
           │  S331
           ▼
     ┌──────────┐
     │  User    │
     │  chart   │
     └──────────┘
```

S337 — Within predicted range?

Yes → No

Adding of warning button — S338

Renewal of measurement data — S336

Extracting of short-term program — S332

Download to health control apparatus — S333

Exercise and measurement — S334

Upload of measurement data — S335

Measurement data

# Fig. 11

Exercise indices,
health control
indices, and
exercise menu for
next duration

303

Health
control
program

304

Exercise indices,
health control
indices, and
exercise menu for
next duration

User
chart

305

Measurement
data in past

User ID

| User ID table / | 320 |
| User ID |

Download

Personal data | 321

User ID

322

Exercise indices,
health control
indices, and
exercise menu

UPDATE

Measurement
data

# Fig. 12

Exercise indices,
health control
indices, and
exercise menu for
next duration

304

303

User
chart

Health
control
program

305    323

Measurement
data

UPDATE

127

101

Exercise indices,
health control
indices, and
exercise menu for
next duration

Measurement
data

# Fig. 13

```
+-----------------------------------------+
| Consumption calorimeter                 |
| main body                               |
|  +------------------------------------+ |        16              17
|11| Heart rate detecting               | |    +----------+   +----------+
|  | means                              | |    | Body     |   | Body     |
|  |  +-------------------------------+ | |    | temperature  | motion   |
|13|  | Sensing means                 | | |    | detecting|   | detecting|
|  |  +-------------------------------+ | |    | means    |   | means    |
|14|  | A/D converting means          | | |    +----+-----+   +-----+----+
|  |  +-------------------------------+ | |         |               |
|15|  | Frequency processing          | | |         v               v
|  |  | means                         | | |    +-------------------------+
|  |  +-------------------------------+ | |    | Correlation relation    |
|  +------------------------------------+ |    | storing means           |
|              |                          |    +-------------------------+
|              |                          |                    18
|  12          |                          |
|   +----------v----------------------+   |
|   | Calorie consumption             |   |
|   | calculating means               |   |
|   +----------------+----------------+   |                       19
|                    |                    |                   +----------+
+--------------------|--------------------+                   | Storing  |
                     |                                        | means    |
   20                |                                        +----------+
    +----------------v----------------+
    | Notifying means                 |
    +---------------------------------+
```

# Fig. 14

# Fig. 15

# Fig. 16-1

```
┌─────────────────────────┐
│        Power ON         │
└─────────────────────────┘
            │  └1601
            ▼
┌─────────────────────────┐              ┌─────────────────────────┐
│ Transmission of target  │              │  Reception of target    │
│ person ID to information│─────────────▶│  person ID              │
│ server                  │              └─────────────────────────┘
└─────────────────────────┘                          │  §1603
            └1602                                     ▼
                                          ┌─────────────────────────┐
                                          │  Searching of target    │
                                          │  person chart           │
                                          └─────────────────────────┘
                                                      │  §1604
                                                      ▼
                                          ┌─────────────────────────┐
                                          │  Extracting of vital    │
                                          │  signs item to be       │
                                          │  monitored              │
                                          └─────────────────────────┘
                                                      │  §1605
                                                      ▼
                                          ┌─────────────────────────┐
                                          │  Transmission of        │
                                          │  vital signs item to    │──▶(A)
                                          │  program server         │
                                          └─────────────────────────┘
                                                      §1606
```

```
      §1610
┌─────────────────────────┐
│  Reception of program   │◀──────────────────────────────────◀(B)
│  and/or data            │
└─────────────────────────┘
            │  §1611
            ▼
┌─────────────────────────┐
│  Notifying of           │
│  vital signs item to be │
│  monitored to target    │
│  person                 │
└─────────────────────────┘
            │  §1612
            ▼
┌─────────────────────────┐
│  Setting-up of program  │
│  and/or data            │
└─────────────────────────┘
            │  §1613
            ▼
┌─────────────────────────┐
│  Operation of program   │
│  according to operation │
│  data                   │
└─────────────────────────┘
            │  §1614
            ▼
┌─────────────────────────┐
│  Acquiring of vital     │
│  signs                  │
└─────────────────────────┘
            │  §1615
            ▼
┌─────────────────────────┐              ┌─────────────────────────┐
│  Transmission of vital  │              │  Reception of           │
│  signs to informat-     │─────────────▶│  vital signs            │
│  ion server             │              └─────────────────────────┘
└─────────────────────────┘                          │  §1616
                                                      ▼
                                          ┌─────────────────────────┐
                                          │  Renewal of contents of │
                                          │  chart                  │
                                          └─────────────────────────┘
                                                      │  §1617
                                                      ▼
                                          ╭─────────────────────────╮
                                          │          End            │
                                          ╰─────────────────────────╯
```

# Fig. 16-2

```
                                        ⌇1607
              ┌─────────────────────────┐
         Ⓐ⤳→  │ Reception of organism   │
              │ information item        │
              └─────────────────────────┘
                            │    ⌇1608
                            ↓
              ┌─────────────────────────┐
              │ Extracting of program   │
              │ and/or data             │
              └─────────────────────────┘
                            │    ⌇1609
                            ↓
              ┌─────────────────────────┐
         Ⓑ◄── │ Transmission of program │
              │ and/or data to terminal │
              └─────────────────────────┘
```

# Fig. 17-1

```
┌────────────────────────┐        ┌────────────────────────┐
│   Start of operation   │        │   Start of operation   │
└───────────┬────────────┘        └───────────┬────────────┘
            │                                 ↓ ç1701
            ↓                     ┌────────────────────────┐
┌────────────────────────┐        │  Searching of target   │
│         Ready          │        │     person chart       │
└───────────┬────────────┘        └───────────┬────────────┘
            │         ↖1700                    ↓ ç1702
            │                     ┌────────────────────────┐
            │                     │ Extracting of vital    │
            │                     │   signs    item to be  │
            │                     │ monitored              │
            │                     └───────────┬────────────┘
            │                                 ↓ ç1703
            │                     ┌────────────────────────┐
            │                     │ Transmission of vital  │──→(C)
            │                     │   signs    item to     │
            │                     │ program server         │
            │          ç1610      └────────────────────────┘
            ↓                                        (D)
┌────────────────────────┐◄──────────────────────────
│ Reception of program   │
│ and/or data            │
└───────────┬────────────┘
            ↓ ç1611
┌────────────────────────┐
│ Notifying of vital     │
│   signs    item to be  │
│ monitored to target    │
│ person                 │
└───────────┬────────────┘
            ↓ ç1612
┌────────────────────────┐
│ Setting-up of program  │
│ and/or data            │
└───────────┬────────────┘
            ↓ ç1613
┌────────────────────────┐
│ Operation of program   │
│ according to operation │
│ data                   │
└───────────┬────────────┘
            ↓ ç1614
┌────────────────────────┐
│ Acquiring of vital     │
│   signs                │
└───────────┬────────────┘
            ↓ ç1615                              ç1616
┌────────────────────────┐        ┌────────────────────────┐
│ Transmission of vital  │───────→│ Reception of vital     │
│   signs    to informat-│        │ signs                  │
│ ion server             │        └───────────┬────────────┘
└────────────────────────┘                    ↓ ç1617
                                   ┌────────────────────────┐
                                   │ Renewal of contents of │
                                   │ chart                  │
                                   └───────────┬────────────┘
                                               ↓
                                   ┌────────────────────────┐
                                   │          End           │
                                   └────────────────────────┘
```

# Fig. 17-2

```
                                    ⌇1607
              ┌──────────────────────────┐
         C    │  Reception of vital      │
              │    signs       item      │
              └──────────────────────────┘
                            │   ⌇1608
                            ▼
              ┌──────────────────────────┐
              │ Extracting of program    │
              │ and/or data              │
              └──────────────────────────┘
                            │   ⌇1609
                            ▼
              ┌──────────────────────────┐
         D    │ Transmission of program  │
              │ and/or data to terminal  │
              └──────────────────────────┘
```

# Fig. 18

```
          ┌────────────────────────┐
          │   Start of operation   │
          └────────────────────────┘
                      │
    ┌─────────────────┼──────────────────────────────────┐
    │                 ▼                                   │
    │   ┌──────────────────────────┐                      │
    │   │ Health control apparatus │  ～1801              │
    │   │ carries out inspection   │                      │
    │   └──────────────────────────┘                      │
    │                 │                                    │
    │                 ▼                                    │
    │   ┌──────────────────────────┐                      │
    │   │ Inspection item judgment │  ～1802              │
    │   │ server managing means judges │                  │
    │   │ inspection result        │                      │
    │   └──────────────────────────┘                      │
    │                 │                                    │
    │                 ▼          1803                      │
    │            ╱─────────────╲                           │
    │           ╱ Is inspection ╲  No                      │
    │           ╲ to be continued?╱──────┐                 │
    │            ╲───────────────╱       ▼    1804         │
    │                 │ Yes      ┌──────────────────┐      │
    │      1805       │          │ Inspection is    │      │
    │            ╱────▼────╲     │ terminated       │      │
    │           ╱ Is inspection╲ └──────────────────┘      │
    │           ╲ item to be   ╱  No                1808   │
    │            ╲ changed?    ╱────────┐   ┌────────────┐ │
    │             ╲──────────╱          │   │ Inspection is│ │
    │   1806          │ Yes             │   │ continued by │ │
    │        ┌────────▼────────┐        │   │ same method  │ │
    │        │ Inspection item │        │   └────────────┘ │
    │        │ is changed      │        │        ▲         │
    │        └─────────────────┘        ▼   1807           │
    │                 │            ╱──────────╲             │
    │                 │           ╱ Is inspection╲  No      │
    │                 │           ╲ method to be ╱──────────┤
    │                 │            ╲ changed?   ╱           │
    │                 │             ╲─────────╱             │
    │                 │              │ Yes   1809           │
    │                 │        ┌─────▼──────────┐           │
    │                 │        │ Inspection method│         │
    │                 │        │ is changed      │          │
    │                 │        └─────────────────┘          │
    └─────────────────┴──────────────┴────────────────────┘
```